# EUROPEAN PATENT APPLICATION

(11) **EP 4 799 564 A1**
(43) Date of publication of application: **02.09.2026**
(21) Application number: 25211498.8
(22) Date of filing: 27.10.2025
(51) Int. Cl.: A61B 5/145

(54) **APPLICATOR AND APPLICATOR ASSEMBLY**

(30) Priority: 07.11.2024 KR 20240157183; 14.01.2025 KR 20250005205
(71) Applicant: i-Sens, Inc., Seoul 06646 (KR)
(72) Inventor: CHOI, Hyun Ho, 06646 Seoul (KR); SEONG, Yul Min, 06646 Seoul (KR); CHAE, Kyung Chul, 06646 Seoul (KR)
(74) Representative: Isarpatent

(57) **Abstract**

According to one aspect of the present invention, an applicator and an applicator assembly capable of stably attaching a wearable unit to skin while inserting a transcutaneous sensor into an accurate subcutaneous position may be provided.

## Description

### BACKGROUND OF THE INVENTION

### Field of the Invention

The present invention relates to an applicator and an applicator assembly, and more specifically, relates to an applicator and an applicator assembly for inserting a transcutaneous sensor that detects biometric information into subcutaneous tissue.

### Description of the Related Art

Chronic diseases such as diabetes mellitus require continuous management. Diabetes mellitus is characterized by having almost no subjective symptoms in an early stage, but when diabetes mellitus progresses, characteristic symptoms such as polydipsia, polyphagia, polyuria, weight loss, general malaise, skin pruritus, cases in which wounds on hands and feet do not heal and last long appear, and the like. When diabetes mellitus progresses further, complications may occur which advance to visual impairment, hypertension, nephropathy, stroke, periodontal disease, muscle cramps and neuralgia, gangrene, and the like. **In** order to diagnose such diabetes mellitus and to manage such that progression to complications does not occur, systematic blood glucose measurement and treatment need to be performed in parallel.

Patients with diabetes mellitus or persons in whom glucose levels at a reference value or more is detected in blood although progression to diabetes mellitus has not occurred, require continuous and periodic blood glucose measurement for management of diabetes mellitus or prevention of progression to diabetes mellitus.

Currently, a blood sampling method in which blood is collected from a position such as a fingertip and the like, and blood glucose is measured in a unit of a single time constitutes a mainstream of blood glucose measurement methods. However, in the case of the blood sampling blood glucose measurement method, due to pain accompanied at the time of blood sampling, inconvenience generated therefrom, and the like, there is a limitation in continuous and periodic blood glucose measurement.

Recently, in order to overcome the limitation of the blood sampling blood glucose measurement method, a blood glucose measurement system which measures blood glucose continuously and periodically by inserting a transcutaneous sensor into subcutaneous tissue has been developed and is being used. The blood glucose measurement system using the transcutaneous sensor is generally configured to include a wearable unit provided to be attachable to a human body including the transcutaneous sensor, an applicator which is provided to be coupled to the wearable unit in advance so as to attach the wearable unit to the human body at the same time as inserting the transcutaneous sensor into the subcutaneous tissue, and a receiving unit which processes information received from the wearable unit.

Meanwhile, in terms of hygiene, infection prevention, and the like, the wearable unit is generally provided to be discarded after a single wearing, and the applicator is also generally provided to be discarded after a single firing. That is, when the transcutaneous sensor is not inserted into an accurate subcutaneous position or the wearable unit is not normally attached to the skin, the corresponding wearable unit and the applicator need to each be discarded regardless of their respective usage lifetimes. Therefore, in order to improve the convenience of use and economic efficiency of the blood glucose measurement system using the transcutaneous sensor, there is required an applicator which may insert the transcutaneous sensor into an accurate subcutaneous position and may also attach the wearable unit stably to the skin.

### SUMMARY OF THE INVENTION

One aspect of the present invention is to provide an applicator and an applicator assembly capable of stably attaching a wearable unit to skin while inserting a transcutaneous sensor into an accurate subcutaneous position.

The object of the present invention is not limited to the above description. A person with ordinary skill in the art to which the present invention pertains will have no difficulty in readily understanding additional objects of the present invention from the overall contents of the present specification.

The present invention is defined by the independent claims. Further aspects of the present invention are outlined in the dependent claims.

An applicator, according to one aspect of the present invention, may comprise: a body housing having an interior in which a movement space is formed; a sensor unit carrier provided to be movable in the movement space; a bridge pressing portion provided in one of the body housing or the sensor unit carrier; and a bridge provided in the other one of the body housing or the sensor unit carrier, and disposed at a position corresponding to the bridge pressing portion, in which the bridge may be in contact with the bridge pressing portion to restrict movement of the sensor unit carrier from an initial position to an insertion position.

An applicator, according to one aspect of the present invention, may comprise: a body housing having an interior in which a movement space is formed; a sensor unit carrier provided to be movable in the movement space; a bridge pressing portion provided in one of the body housing or the sensor unit carrier; and a bridge provided in the other one of the body housing or the sensor unit carrier, disposed at a position corresponding to the bridge pressing portion, and configured to restrict movement of the sensor unit carrier from an initial position to an insertion position.

The body housing may comprise a column disposed to form the movement space in the interior of the body housing and provided with the bridge pressing portion at an end portion thereof.

The sensor unit carrier may comprise: a sensor unit carrier body of which at least a portion is accommodated in the movement space and is provided to move along the movement space; an extension arm extending from a side end of the sensor unit carrier body and disposed at an outside of the column; and the bridge having one end connected to the sensor unit carrier body and the other end connected to the extension arm, and disposed at a position facing the bridge pressing portion.

When the sensor unit carrier is positioned at the initial position, the bridge may be in contact with the bridge pressing portion or be positioned at a position spaced apart from the bridge pressing portion.

The sensor unit carrier may move in the movement space along a first direction from the initial position to the insertion position.

When the sensor unit carrier is positioned at the insertion position, at least a portion of the bridge may be positioned at a position overlapping at least a portion of the bridge pressing portion.

An applicator, according to one aspect of the present invention, may comprise: a body housing having an interior in which a movement space is formed along a first direction; a sensor unit carrier provided to be relatively movable with respect to the body housing in the movement space; a bridge pressing portion provided in one of the body housing or the sensor unit carrier; and a bridge disposed at a position contactable with the bridge pressing portion in the other one of the body housing or the sensor unit carrier, in which movement restriction of the sensor unit carrier in the first direction may be released by shape deformation of the bridge due to pressing of the bridge pressing portion.

An applicator, according to one aspect of the present invention, may comprise: a body housing having an interior in which a movement space is formed along a first direction; a sensor unit carrier provided to be relatively movable with respect to the body housing in the movement space; a bridge pressing portion provided in one of the body housing or the sensor unit carrier; and a bridge configured to restrict movement of the sensor unit carrier and disposed at a position contactable with the bridge pressing portion in the other one of the body housing or the sensor unit carrier, in which movement restriction of the sensor unit carrier in the first direction may be released by shape deformation of the bridge due to pressing of the bridge pressing portion.

The bridge may comprise a vulnerable portion in which shape deformation is concentrated compared to other regions when pressure is applied by the bridge pressing portion.

The body housing may comprise a column disposed to form the movement space in the interior of the body housing and provided with the bridge pressing portion at an end portion thereof.

The sensor unit carrier may comprise: a sensor unit carrier body of which at least a portion is accommodated in the movement space and is provided to move along the movement space; an extension arm extending from a side end of the sensor unit carrier body and disposed at an outside of the column; and the bridge having one end connected to the sensor unit carrier body and the other end connected to the extension arm, and disposed at a position facing the bridge pressing portion, and the vulnerable portion may be formed at a position closer to one surface of the sensor unit carrier body to which the bridge is connected compared to a leading end portion of the bridge pressing portion.

A carrier slit formed by incision along the first direction may be provided at a leading end portion of the column adjacent to the bridge pressing portion.

An extension arm guide groove may be formed to be recessed along the first direction in a shape corresponding to the carrier slit on one surface of an extension arm connection portion disposed between the sensor unit carrier body and the extension arm.

As the sensor unit carrier moves along the first direction, the extension arm guide groove may be introduced into the carrier slit so that movement of the sensor unit carrier in the first direction is guided.

The vulnerable portion may be formed to have a smaller thickness compared to the bridge.

The bridge pressing portion may be provided with at least one inclined surface and be disposed at the end portion of the column in a shape in which a cross-section decreases toward the bridge.

The bridge may be disposed in a direction intersecting the first direction, and the bridge pressing portion may be disposed along the first direction.

Movement restriction of the sensor unit carrier in the first direction may be released by cutting of the bridge.

The applicator may further comprise: a handle housing connected to the body housing so that one end thereof is in contact with the sensor unit carrier and moves together with the sensor unit carrier, in which movement restriction of the handle housing in the first direction may be released only when the bridge is deformed by application of a pressure exceeding a reference pressure to the handle housing.

An applicator assembly, according to one aspect of the present invention, may comprise: a transmission unit configured to be delivered to a detection position; a body housing having an interior in which a movement space is formed along a first direction, the transmission unit being detachably fixed to one end thereof; a sensor unit carrier provided to move along the movement space; a sensor unit comprising a transcutaneous sensor member capable of detecting biometric information in a subcutaneous tissue of a human body, and provided to move together with the sensor unit carrier in the movement space along the first direction to be coupled to the transmission unit; a bridge pressing portion provided in one of the body housing or the sensor unit carrier; and a bridge disposed at a position corresponding to the bridge pressing portion in the other one of the body housing or the sensor unit carrier, and provided to be deformed or cut only when a pressure applied from the bridge pressing portion exceeds a reference pressure, in which movement restriction of the sensor unit carrier in the first direction may be released by deforming or cutting of the bridge.

The bridge may be provided in a shape extending along a direction intersecting the first direction.

The bridge pressing portion may be disposed along a direction parallel to the first direction.

According to one aspect of the present invention, an applicator and an applicator assembly capable of stably attaching a wearable unit to skin while inserting a transcutaneous sensor into an accurate subcutaneous position may be provided.

An applicator, according to one aspect of the present invention, may comprise: a body housing forming a space capable of accommodating a first unit; and a unit carrier provided to be movable within the space.

The first unit may include a sensor member configured for obtaining a glucose data.

An applicator, according to one aspect of the present invention, comprising: a body housing in which an accommodation portion capable of accommodating a first unit is provided at one end thereof and a movement space connected to the accommodation portion is formed; a first unit carrier configured to move in the movement space.

The housing may include a unit support portion provided on one side of the receiving portion to limit movement of the first unit.

The unit support portion is pressed by one end of the first unit carrier as the first unit carrier moves in a direction becoming closer to the accommodation portion, and is torsionally moved from a restriction position in which at least one end thereof contacts the first unit accommodated in the accommodation portion to a release position in which at least one end thereof moves away from the first unit accommodated in the accommodation portion as the unit support portion is pressed by the first unit carrier.

An applicator assembly, according to one aspect of the present invention, may comprise: an applicator configured for ejecting a sensor member; and a cap separably coupled to one end of an applicator.

An applicator, according to one aspect of the present invention, may comprise: a body housing forming an internal space capable of accommodating a first unit having a sensor member; and a handle housing disposed to overlap at least a portion of the body housing.

According to one aspect of the present invention, an applicator assembly may include a sensor member configured to obtain an analyte data.

According to one aspect of the present invention, a sensor member may be a glucose sensor configured to obtain a glucose data.

According to one aspect of the present invention, at least a portion of a sensor member may be electrically coupled with a circuit board.

According to one aspect of the present invention, at least a portion of the sensor member may have a surface facing towards a skin or extend towards the skin.

According to one aspect of the present invention, an applicator and an applicator assembly capable of improving safety of an invasive medical device.

According to one aspect of the present invention, an applicator and an applicator assembly capable of inserting a transcutaneous sensor member into subcutaneous tissue when a predetermined force or more is applied to the applicator.

According to one aspect of the present invention, an applicator and an applicator assembly capable of penetrating the skin at a speed and with a force suitable for a needle insertion.

The effects of the present invention are not limited to the above description, and may include matters that can be reasonably inferred from the following description by a person with ordinary skill in the art to which the present invention pertains.

### BRIEF DESCRIPTION OF THE DRAWINGS

FIG. 1 is a perspective view illustrating an exemplary embodiment of an applicator assembly.
FIG. 2 is a perspective view illustrating an exemplary embodiment of the applicator assembly with a cap removed.
FIG. 3 is a perspective view illustrating an exemplary embodiment of a wearable unit.
FIG. 4 is a conceptual view illustrating an exemplary embodiment of the wearable unit attached to a human body and a remote terminal.
FIG. 5 is a perspective view illustrating an exemplary embodiment of a sensor unit in a state in which needles are coupled.
FIG. 6 is a perspective view illustrating an exemplary embodiment of a coupling process of the sensor unit and a transmission unit.
FIGS. 7 and 8 are exploded perspective views illustrating an exemplary embodiment of the applicator assembly with the cap removed.
FIG. 9 is a perspective view illustrating an exemplary embodiment of a handle housing.
FIG. 10 is a bottom view illustrating an exemplary embodiment of the handle housing.
FIG. 11 is a cross-sectional view illustrating the handle housing of FIGS. 9 and 10 cut in an A-A' direction.
FIG. 12 is a perspective view and a partially enlarged view illustrating an exemplary embodiment of a body housing.
FIG. 13 is a bottom view illustrating an exemplary embodiment of the body housing.
FIG. 14 is a plan view illustrating an exemplary embodiment of the body housing.
FIG. 15 is a cross-sectional view illustrating the body housing cut along B-B' of FIG. 14.
FIG. 16 is a partial cross-sectional perspective view illustrating the body housing cut along C-C' of FIG. 14.
FIGS. 17 and 18 are perspective views illustrating an exemplary embodiment of a sensor unit carrier.
FIG. 19 is a perspective view exemplarily illustrating a coupling relationship of a needle carrier.
FIG. 20 is a perspective view illustrating an exemplary embodiment of the needle carrier.
FIG. 21 is a front view exemplarily illustrating a coupling relationship of the needle carrier and the sensor unit carrier.
FIG. 22 is an exploded perspective view illustrating an exemplary embodiment of the cap.
FIG. 23 is a cross-sectional view illustrating the cap cut along an E-E' direction in FIG. 22.
FIGS. 24 to 26 are partially enlarged cross-sectional views illustrating an exemplary embodiment of the applicator assembly to which the cap is applied.
FIG. 27 is a partial cross-sectional view illustrating an exemplary embodiment of a state in which the sensor unit carrier is coupled to the body housing during an assembly process of the applicator assembly.
FIGS. 28 and 29 are partial cross-sectional views illustrating an exemplary embodiment of a process of coupling the handle housing to the body housing to which the sensor unit carrier is coupled during an assembly process of the applicator assembly.
FIG. 30 is an enlarged cross-sectional view illustrating an exemplary embodiment of a state in which a leading end portion of a fixing protrusion portion is inserted into a fixing groove during an assembly process of the applicator assembly.
FIG. 31 is an enlarged cross-sectional view illustrating an exemplary embodiment of a state in which the leading end portion of the fixing protrusion portion is discharged from the fixing groove during an assembly process of the applicator assembly.
FIGS. 32 to 34 are partial cross-sectional views sequentially illustrating an exemplary embodiment of a state in which the sensor unit carrier moves from an initial position to an insertion position during an operation process of the applicator assembly.
FIG. 35 is a partial cross-sectional view illustrating an exemplary embodiment of a state in which the needle carrier moves to a retracted position during an operation process of the applicator assembly.
FIG. 36 is a partial cross-sectional view illustrating an exemplary embodiment of a state before a bridge is cut during an operation process of the applicator assembly.
FIG. 37 is a partial cross-sectional view illustrating an exemplary embodiment of a state in which the bridge is cut during an operation process of the applicator assembly.
FIGS. 38 and 39 are partial cross-sectional views illustrating an exemplary embodiment of a deformable bridge and a bridge pressing portion.
FIG. 40 is a cross-sectional view and a partial enlarged cross-sectional view for explaining an exemplary embodiment regarding a positional relationship between a first movement restriction portion and a second movement restriction portion in a state before operation of the applicator assembly.
FIGS. 41(a) to 41(c) are sequentially illustrated partial enlarged cross-sectional views for explaining an exemplary embodiment regarding a positional relationship between the first movement restriction portion and the second movement restriction portion during an operation process of the applicator assembly.
FIGS. 42 to 44 are sequentially illustrated cross-sectional views for explaining an exemplary embodiment of pressing and pressing release of a grip arm protrusion portion during an operation process of the applicator assembly.
FIGS. 45 and 46 are sequentially illustrated cross-sectional views for explaining an exemplary embodiment of movement constraint and movement constraint release of a transmission unit by a transmission unit support portion during an operation process of the applicator assembly.

### DETAILED DESCRIPTION OF THE INVENTION

Hereinafter, preferred embodiments of an applicator and an applicator assembly according to one aspect of the present invention will be described in more detail with reference to the accompanying drawings. Embodiments of the present invention may be modified into various forms, and the scope of the present invention should not be construed as being limited to the embodiments described below. These embodiments are provided to more specifically describe the present invention to those skilled in the technical field to which the present invention pertains. Accordingly, a shape of each element illustrated in the drawings may be emphasized or exaggerated for a clearer description.

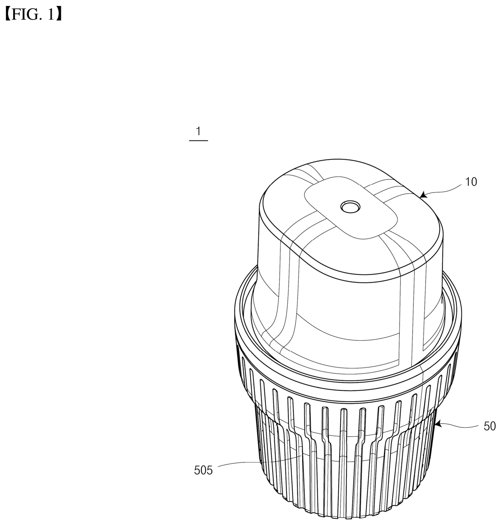

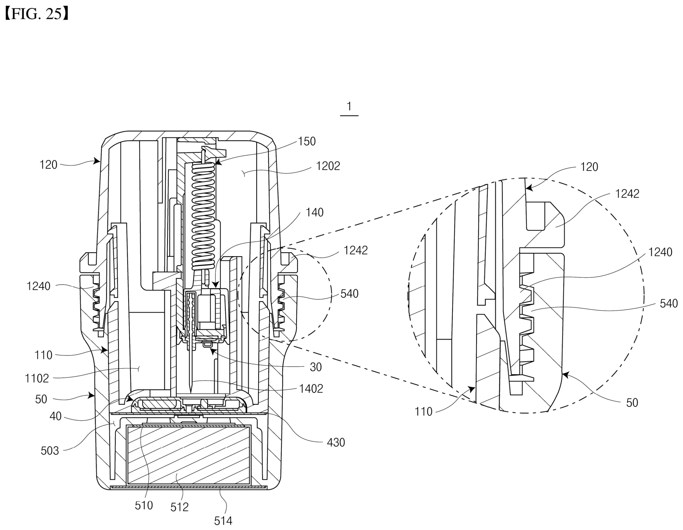

Hereinafter, with reference to FIGS. 1 to 26, the applicator and the applicator assembly will be described in more detail.

### Applicator assembly

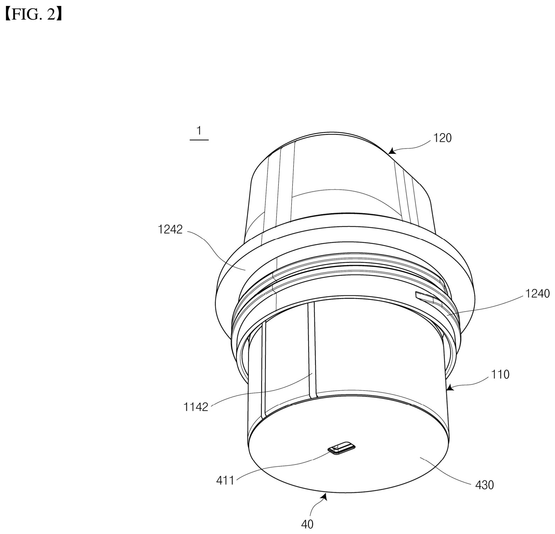

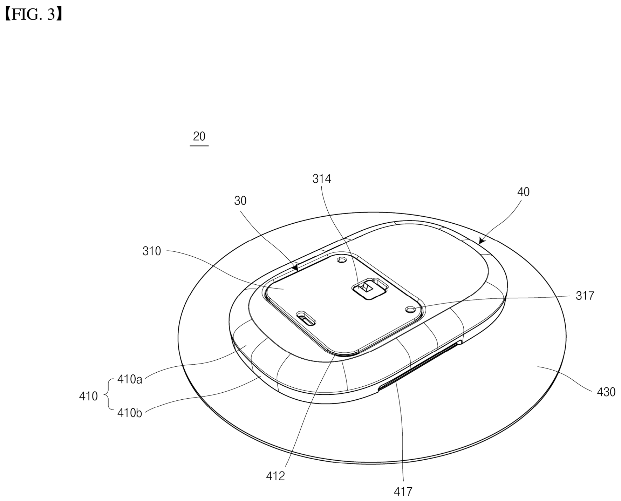

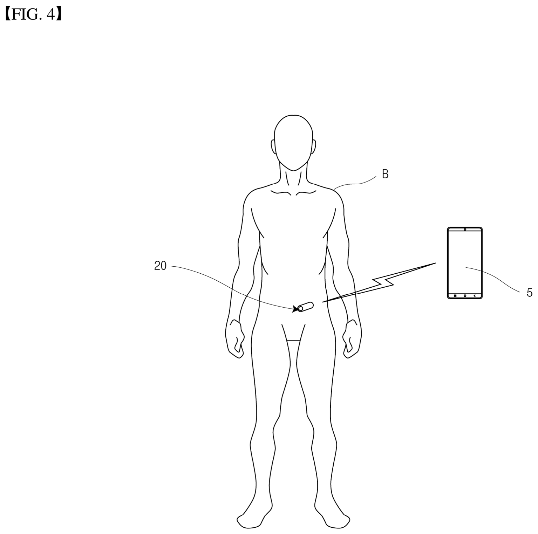

FIG. 1 is a perspective view illustrating an exemplary embodiment of an applicator assembly 1, and FIG. 2 is a perspective view illustrating an exemplary embodiment of the applicator assembly 1 with a cap 50 removed. FIG. 3 is a perspective view illustrating an exemplary embodiment of a wearable unit 20, and FIG. 4 is a conceptual view illustrating an exemplary embodiment of the wearable unit 20 attached to a human body B and a remote terminal 5.

The applicator assembly 1 may include the wearable unit 20 and an applicator 10 that is assembled in advance together with the wearable unit 20 and provided. At one end of the applicator 10, the cap 50 may be detachably disposed. By the cap 50 disposed at one end of the applicator 10, arbitrary firing of the applicator 10 may be prevented, and external contaminants or moisture may be prevented from being introduced into an interior of the applicator assembly 1.

The wearable unit 20 may include a sensor member 330. The sensor member may be a transcutaneous sensor member. The transcutaneous sensor member may be an invasive sensor or a non-invasive sensor including optical sensor.

The transcutaneous sensor member 330 inserted into subcutaneous tissue of the human body B and detecting biometric information. Although the biometric information detected by the transcutaneous sensor member 330 is various, the preferred biometric information detected by the transcutaneous sensor member 330 may be glucose concentration, ketone, glycated hemoglobin (HbAlc), Fructosamine, 1,5-Anhydroglucitol, other blood-based markers or a combination thereof.

The applicator 10 may be used to deliver the wearable unit 20 onto a detection position on skin such that an end portion of the transcutaneous sensor member 330 included in the wearable unit 20 is inserted into the subcutaneous tissue of the human body B. At one end of the wearable unit 20, an adhesive member 430 may be provided, and the wearable unit 20 may be maintained at the detection position for a predetermined period of time by the adhesive member 430.

The wearable unit 20 may preferably be provided in the applicator 10 such that an adhesive surface of the adhesive member 430 is exposed to the exterior in a state in which the cap 50 is removed from the applicator assembly 1. The detection position is not limited to a specific position on the human body B, but in terms of convenience in daily life, it may be preferable that the wearable unit 20 is attached on skin of a site of the human body B such as an upper arm, a thigh, an abdomen, or the like.

The wearable unit 20 may be attached to the skin of the human body B, detect biometric information, and wirelessly transmit detected biometric information data to an external terminal 5. A wireless transmission method is not particularly limited, and wireless transmission methods such as Bluetooth (BT), Bluetooth Low Energy (BLE), Near Field Communication (NFC), Radio Frequency Identification (RFID), and the like may be applied.

The external terminal 5 is also not particularly limited as long as it is a device capable of receiving and processing data, and a mobile terminal, a dedicated medical device, a PC, a server, and the like may be applied. As a non-limiting example, the wearable unit 20 may continuously or periodically detect glucose concentration of the human body B and transmit glucose concentration data to the external terminal 5.

### Wearable unit

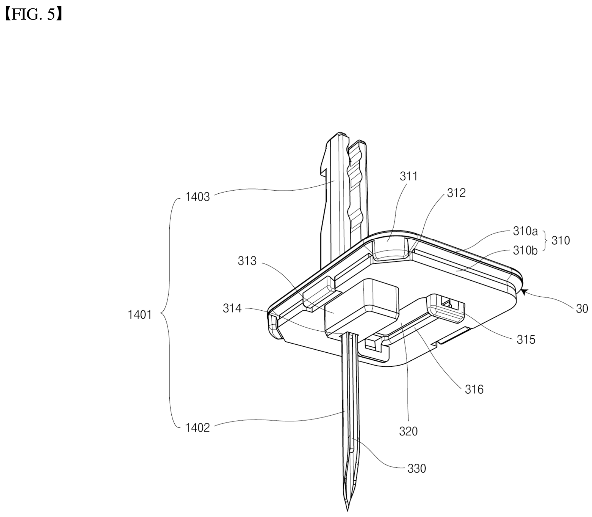

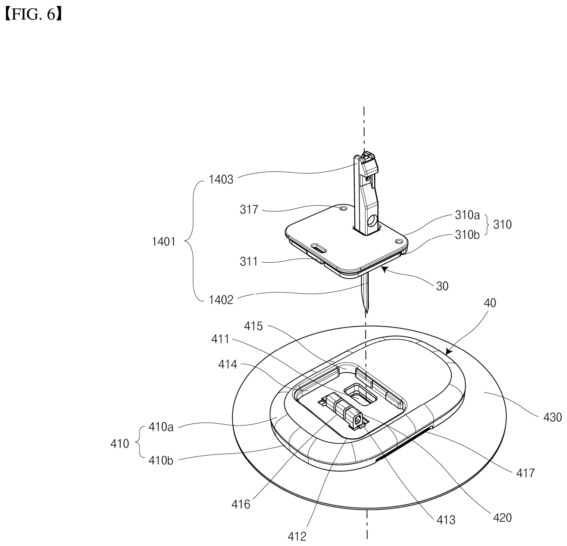

FIG. 5 is a perspective view illustrating an exemplary embodiment of a sensor unit 30 in a state in which needles 1401 are coupled, and FIG. 6 is a perspective view illustrating an exemplary embodiment of a coupling process of the sensor unit 30 and a transmission unit 40.

The wearable unit 20 may include the sensor unit 30 and the transmission unit 40. The wearable unit 20 may be disposed in an interior of the applicator 10 in an integrated form in which the sensor unit 30 and the transmission unit 40 are coupled. In this case, in a firing process of the applicator 10, the wearable unit 20 in the integrated form in which the sensor unit 30 and the transmission unit 40 are coupled may be attached to skin of the human body B.

Meanwhile, it may include a case in which the sensor unit 30 and the transmission unit 40 are disposed in the interior of the applicator 10 in a state separated from each other. In this case, in a process in which the transcutaneous sensor member 330 is inserted into subcutaneous tissue, the sensor unit 30 and the transmission unit 40 may be coupled and disposed at a detection position. It may include all of cases in which the sensor unit 30 and the transmission unit 40 are coupled before the transcutaneous sensor member 330 is inserted into the subcutaneous tissue, or the sensor unit 30 and the transmission unit 40 are coupled simultaneously with insertion of the transcutaneous sensor member 330 into the subcutaneous tissue, or the sensor unit 30 and the transmission unit 40 are coupled after the transcutaneous sensor member 330 is inserted into the subcutaneous tissue.

When the wearable unit 20 is disposed in the interior of the applicator 10 in a state in which the sensor unit 30 and the transmission unit 40 are separated, in an insertion process of the transcutaneous sensor member 330 into the subcutaneous tissue, the sensor unit 30 may relatively move with respect to the transmission unit 40, so that coupling of the sensor unit 30 and the transmission unit 40 may be performed. That is, in a state in which the transmission unit 40 is positioned at the detection position, the sensor unit 30 may move toward the transmission unit 40 so that insertion of the transcutaneous sensor member 330 into the subcutaneous tissue may be performed.

When the sensor unit 30 moves toward the transmission unit 40 so that insertion of the transcutaneous sensor member 330 into the subcutaneous tissue is performed, compared to the wearable unit 20 provided in an integrated form, even when a relatively smaller propulsive force is applied to the transcutaneous sensor member 330, the transcutaneous sensor member 330 may be inserted into an accurate position, and pain and discomfort generated in an insertion process of the transcutaneous sensor member 330 into the subcutaneous tissue may be effectively reduced.

The transmission unit 40 may include a transmission unit housing 410 forming an external shape of the transmission unit 40. A first transmission unit housing 410a and a second transmission unit housing 410b may be coupled to each other to form the transmission unit housing 410, and in an interior of the transmission unit housing 410, a battery serving as a power source and an electronic unit transmitting biometric information data may be provided. At a coupling portion of the first transmission unit housing 410a and the second transmission unit housing 410b, a sealing portion for preventing external contaminants or moisture from being introduced into the interior of the transmission unit housing 410 may be provided.

At one end of the first transmission unit housing 410a, a seating groove 412 in which the sensor unit 30 is accommodated and coupled may be formed to be recessed toward an inside of the transmission unit housing 410. The seating groove 412 may be formed in a shape recessed toward the inside of the transmission unit housing 410 from one surface of the first transmission unit housing 410a. The seating groove 412 may preferably be formed in a shape corresponding to a sensor unit housing 310 to be described later. **In** one region of the first transmission unit housing 410a forming a side surface of the seating groove 412, a side surface protrusion portion 414 having a shape protruding toward the seating groove 412 may be provided. The side surface protrusion portion 414 may be divided into a plurality of portions by a division portion 415, and at the division portion 415, a sensor unit housing protrusion portion 311 to be described later may be disposed when the sensor unit is coupled with the transmission unit.

On one surface of the first transmission unit housing 410a forming the seating groove 412, a first connection opening 416 connecting the interior of the transmission unit housing 410 and the exterior may be formed through. A transmission unit connector 420 connected to the electronic unit disposed in the interior of the transmission unit housing 410 may be exposed to the exterior through the first connection opening 416. A shape of the transmission unit connector 420 is not limited to the shape illustrated in the drawings, and as long as the shape is electrically connectable to a connection terminal intended to be connected to the transmission unit connector 420, it is not limited and may be variously modified and applied. It may be preferable that the transmission unit connector 420 is provided in a material capable of conducting electricity, and in terms of contact stability, it may be more preferable that the transmission unit connector 420 is provided in a material or structure having self-elasticity.

At an end of the first transmission unit housing 410a forming a boundary with the first connection opening 416, a fastening latch 413 formed to protrude toward an inside of the seating groove 412 may be provided. The fastening latch 413 may be coupled to a fastening ring 315 to be described later, and the fastening latch 413 may be fastened to the fastening ring 315, thereby assisting in maintaining a firm coupled state of the sensor unit 30 and the transmission unit 40.

An insertion hole 411 may be formed in a shape penetrating the first transmission unit housing 410a and the second transmission unit housing 410b from the inside of the seating groove 412. When the transcutaneous sensor member 330 is inserted into subcutaneous tissue, at least part of a needle body 1402 and at least part of the transcutaneous sensor member 330 may pass through the insertion hole 411 and be inserted into the subcutaneous tissue. After an end portion of the transcutaneous sensor member 330 is inserted into the subcutaneous tissue, the needle body 1402 is discharged from skin through the insertion hole 411, and one end of the transcutaneous sensor member 330 may be maintained in a state inserted into the subcutaneous tissue.

At one end of the second transmission unit housing 410b opposite to one end in which the seating groove 412 is formed, the adhesive member 430 may be provided. By the adhesive member 430 attached to skin, the wearable unit 20 may be maintained at a detection position for a predetermined period of time. The adhesive member 430 may include a first adhesive surface adhered to skin and a second adhesive surface adhered to one end of the second transmission unit housing 410b. For protection of the first adhesive surface before attachment to skin, a protective film may be additionally provided on the first adhesive surface attached to skin. The protective film may be an optional component. In FIG. 6, the adhesive member 430 provided to have a larger area than one end of the second transmission unit housing 410b is illustrated, but a size or a shape of the adhesive member 430 is not necessarily limited thereto. The adhesive member 430 may be provided to have an area corresponding to one end of the second transmission unit housing 410b, or may be provided to have a smaller/identical area than/to one end of the second transmission unit housing 410b. The adhesive member 430 may be separately provided so that a user attaches it to the transmission unit housing 410 in an insertion process of the transcutaneous sensor member 330 into subcutaneous tissue, but may include a case in which the adhesive member 430 is provided to maintain a state in which the adhesive member 430 is pre-attached to the transmission unit housing 410 disposed in an interior of the applicator assembly 1.

A transmission unit housing groove 417 may be provided on a side end of the transmission unit housing 410. For example, on a side end of one end side of the second transmission unit housing 410b facing the adhesive member 430, a transmission unit housing groove 417 formed to be recessed toward the inside of the transmission unit housing 410 may be provided. An end portion of a catching portion 1164 provided in a transmission unit support portion 1160 to be described later may be disposed in an interior of the transmission unit housing groove 417, and in this case, the transmission unit 40 may be effectively prevented from being arbitrarily deviated from the applicator 10.

The sensor unit 30 may include a sensor unit housing 310 forming an external shape of the sensor unit 30, and a transcutaneous sensor member 330 disposed in the interior of the sensor unit housing 310 and provided such that one end thereof is exposed to an exterior of the sensor unit housing 310. When the wearable unit 20 is attached to the human body B, the one end of the transcutaneous sensor member 330 exposed to the exterior of the sensor unit housing 310 may be maintained in a state inserted into subcutaneous tissue, and biometric information may be detected by the transcutaneous sensor member 330 percutaneously inserted into the subcutaneous tissue.

The sensor unit housing 310 may be formed by coupling a first sensor unit housing 310a and a second sensor unit housing 310b to each other. At a coupling portion of the first sensor unit housing 310a and the second sensor unit housing 310b, a sealing portion for preventing external contaminants or moisture from being introduced into the interior of the sensor unit housing 310 may be provided. At a side end portion of the second sensor unit housing 310b, a plurality of concave portions 312 having shapes recessed from a side surface of the second sensor unit housing 310b may be formed spaced apart at predetermined intervals along a circumferential direction of the second sensor unit housing 310b, and on one surface of the first sensor unit housing 310a facing the second sensor unit housing 310b, a sensor unit housing protrusion portion 311 formed to protrude at positions corresponding to the plurality of concave portions 312 may be provided.

The sensor unit housing protrusion portion 311 and the concave portion 312 may be provided to have a shape in which the sensor unit housing protrusion portion 311 is in close contact with the concave portion 312 or the sensor unit housing protrusion portion 311 is capable of pressing the concave portion 312 in a state in which the first sensor unit housing 310a and the second sensor unit housing 310b are coupled. Since the sensor unit housing protrusion portion 311 is maintained in a state of being in close contact with or pressing the concave portion 312 in a state in which the first sensor unit housing 310a and the second sensor unit housing 310b are coupled to each other, the first sensor unit housing 310a and the second sensor unit housing 310b may be maintained in a more firmly coupled state.

Meanwhile, the side surface protrusion portion 414 formed in the transmission unit housing 410 may be provided to have a shape being in close contact with a side end of the second sensor unit housing 310b or capable of pressing the side end of the second sensor unit housing 310b in a state in which the sensor unit 30 and the transmission unit 40 are coupled, and by the close contact between the side surface protrusion portion 414 and the second sensor unit housing 310b or by the pressing of the second sensor unit housing 310b by the side surface protrusion portion 414, the sensor unit 30 and the transmission unit 40 may be maintained in a more firmly fixed state. When a coupled state of the sensor unit 30 and the transmission unit 40 is maintained, the sensor unit housing protrusion portion 311 may be disposed in an interior of the division portion 415 formed at a side end of the seating groove 412, and the sensor unit housing protrusion portion 311 may be in close contact with one surface of the first transmission unit housing 410a forming the division portion 415, or one surface of the first transmission unit housing 410a forming the division portion 415 may press the sensor unit housing protrusion portion 311.

At one end of the second sensor unit housing 310b disposed to face the seating groove 412 during coupling of the sensor unit 30 and the transmission unit 40, a second connection opening 316 connecting an interior of the sensor unit housing 310 and the exterior may be formed through. A sensor unit connector 320 connected to the transcutaneous sensor member 330 may be exposed to the exterior through the second connection opening 316. During coupling of the sensor unit 30 and the transmission unit 40, the sensor unit connector 320 and the transmission unit connector 420 may be electrically in contact with each other, and biometric information data detected by the transcutaneous sensor member 330 may be delivered to an electronic unit provided in the transmission unit 40 through the sensor unit connector 320 and the transmission unit connector 420.

At one end of the second sensor unit housing 310b facing the transmission unit 40, a boss 313 formed to protrude in a shape corresponding to the insertion hole 411 may be provided. **In** the boss 313, a through-hole 314 communicating an interior of the sensor unit housing 310 with the exterior may be formed through, and one end of the transcutaneous sensor member 330 intended to be inserted into subcutaneous tissue may extend from the interior of the sensor unit housing 310 to the exterior of the sensor unit housing 310 through the through-hole 314 and be disposed. The through-hole 314 may be formed to extend through not only the second sensor unit housing 310b but also the first sensor unit housing 310a. The needle body 1402 may be disposed to pass through the through-hole 314 in a state in which the sensor unit 30 and the needle 1401 are coupled. **In** this case, one end of the transcutaneous sensor member 330 extending to the exterior of the sensor unit housing 310 may be disposed in the exterior of the sensor unit housing 310 in a state accommodated in an interior of the needle body 1402. During coupling of the sensor unit 30 and the transmission unit 40, a circumferential side surface of the boss 313 and an inner surface of the transmission unit housing 410 forming the insertion hole 411 may come into contact with each other in a state of being in close contact. Accordingly, not only may one end of the transcutaneous sensor member 330 be inserted into an accurate position, but also the sensor unit 30 and the transmission unit 40 may be maintained in a more firmly coupled state. Meanwhile, in a state in which the sensor unit 30 and the transmission unit 40 are mutually coupled, the circumferential side surface of the boss 313 and the inner surface of the transmission unit housing 410 forming the insertion hole 411 may be maintained in a state of close contact with each other, and in a state in which the wearable unit 20 is attached to the human body B, it is possible to effectively prevent external contaminants or moisture from being introduced toward the sensor unit connector 320 and the transmission unit connector 420.

At an end portion of the second sensor unit housing 310b forming a boundary with the second connection opening 316, the fastening ring 315 formed to protrude along a direction parallel to a protruding direction of the transcutaneous sensor member 330 may be provided. **In** a coupling process of the sensor unit 30 and the transmission unit 40, the fastening latch 413 may be fixed to the fastening ring 315, and by mutual coupling of the fastening latch 413 and the fastening ring 315, the sensor unit 30 and the transmission unit 40 may be maintained in a firmly coupled state. **In** the above, an example is described in which the fastening latch 413 is provided in the first transmission unit housing 410a, and the fastening ring 315 is provided in the second sensor unit housing 310b, but it may include a case in which the fastening latch is provided in the second sensor unit housing 310b, and the fastening ring is provided in the first transmission unit housing 410a. Meanwhile, the fastening means of the sensor unit 30 and the transmission unit 40 are not limited to a latch and a ring, and as long as the fastening means are capable of preventing the sensor unit 30 from being separated from the transmission unit 40 after coupling of the sensor unit 30 and the transmission unit 40, while not interfering with movement of the sensor unit 30 toward the transmission unit 40 in an insertion process of the transcutaneous sensor member 330 into subcutaneous tissue, the fastening means may be variously modified and applied.

On one surface of the first sensor unit housing 310a, a fixing groove 317 may be formed to be recessed, and a leading end portion of a fixing protrusion 1317 formed in a sensor unit carrier 130 to be described later may be inserted into the fixing groove 317 and be disposed. **In** an insertion process of the transcutaneous sensor member 330 into subcutaneous tissue, the sensor unit 30 may move toward the transmission unit 40 together with the sensor unit carrier 130, and during movement of the sensor unit 30, the leading end portion of the fixing protrusion 1317 may maintain a state of being inserted into the fixing groove 317 so that the sensor unit 30 may be stably supported by the sensor unit carrier 130 and move. After insertion of the transcutaneous sensor member 330 into the subcutaneous tissue, in a process in which a user removes the applicator 10 from skin, the fixing protrusion 1317 may be discharged from the fixing groove 317, and thereby, a fixing relationship between the sensor unit carrier 130 and the sensor unit 30 may be released.

### Applicator

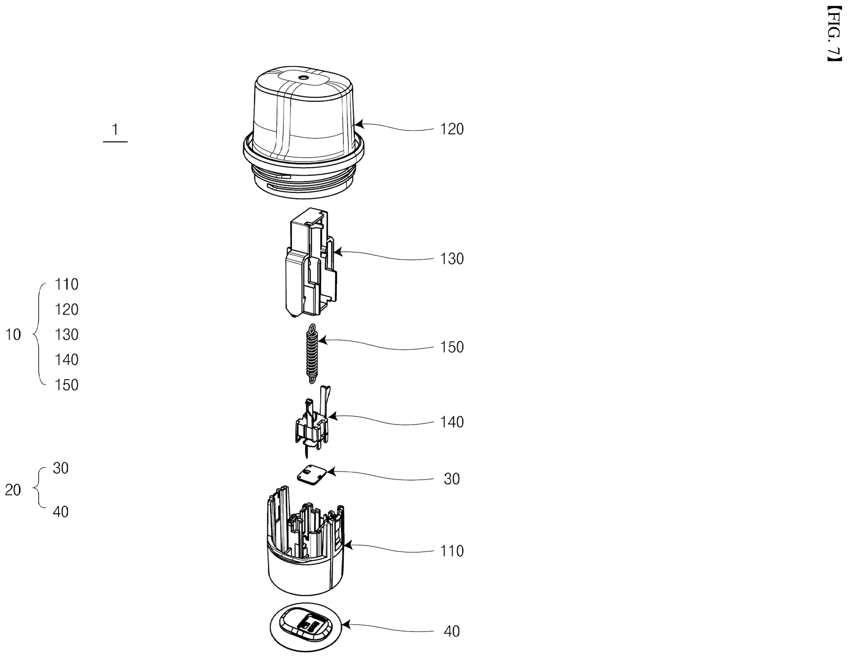

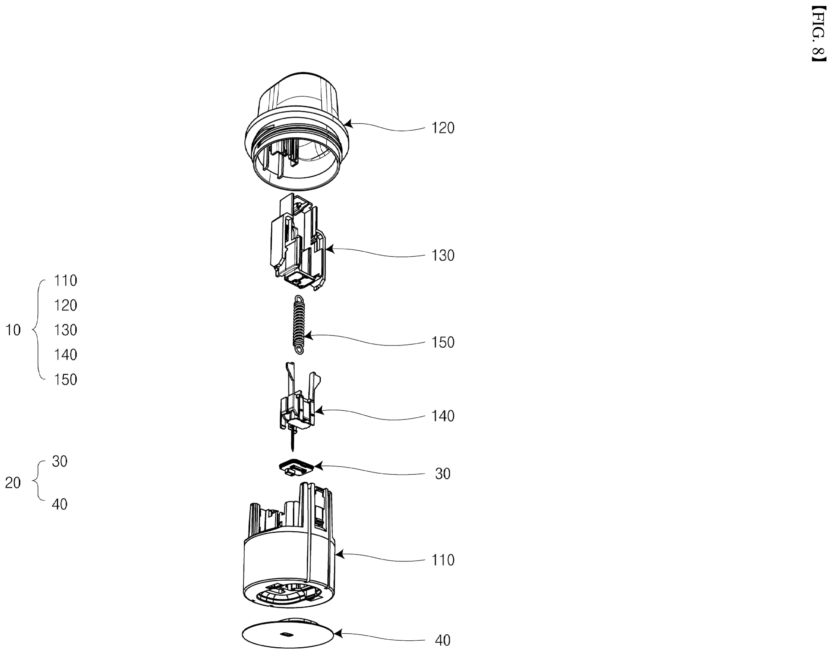

FIGS. 7 and 8 are exploded perspective views illustrating an exemplary embodiment of the applicator assembly 1 with the cap 50 removed. Hereinafter, for convenience of description, a direction generally parallel with an insertion direction of the transcutaneous sensor member 330 is defined as a first direction, and all directions generally perpendicular to the first direction are defined as a second direction, and a specific configuration of the applicator 10 and the applicator assembly 1 is described.

The applicator 10 may be provided to deliver the wearable unit 20 to a detection position on skin. The applicator 10 may include a body housing 110 to which the transmission unit 40 is detachably coupled at one end, a handle housing 120 disposed to relatively move with respect to the body housing 110 along the first direction during insertion of the transcutaneous sensor member 330 into subcutaneous tissue, a sensor unit carrier 130 to which the sensor unit 30 is detachably coupled at one end and which is disposed to move together with the handle housing 120 along the first direction, a needle carrier 140 provided with the needle body 1402 for insertion of the transcutaneous sensor member 330 into subcutaneous tissue and separably and fixedly disposed to the sensor unit carrier 130, and an elastic member 150 having one end and the other end connected to the sensor unit carrier 130 and the needle carrier 140, respectively, and providing driving force such that the needle body 1402 inserted into the subcutaneous tissue is discharged from the human body B.

The applicator assembly 1 may further include, in addition to the above-described applicator 10, the transmission unit 40 separably and fixedly disposed to one end of the body housing 110, the sensor unit 30 separably disposed to one end of the sensor unit carrier 130, and the cap 50 detachably coupled to the handle housing 120 so as to block external exposure of the body housing 110 in which the transmission unit 40 is disposed.

### Handle housing

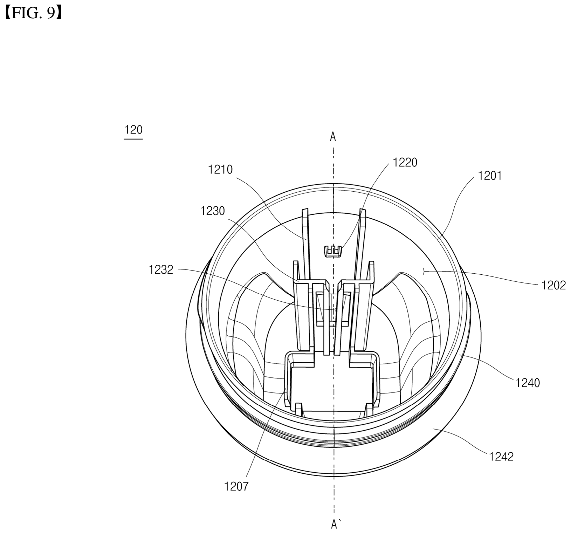

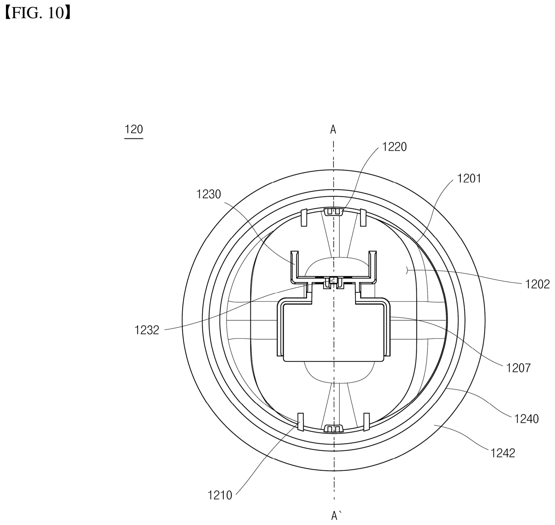

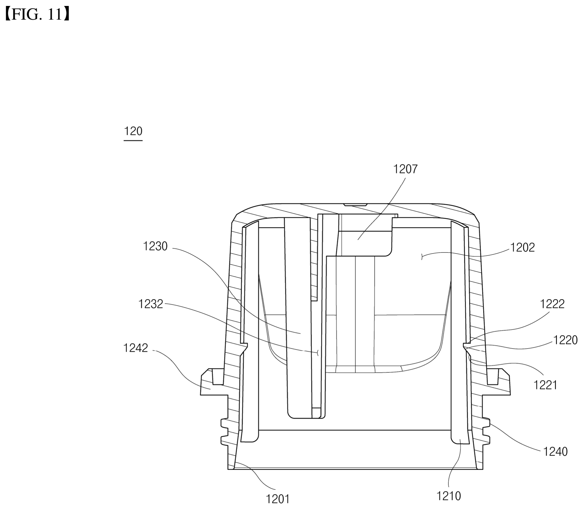

FIGS. 9 and 10 are a perspective view and a bottom view illustrating an exemplary embodiment of the handle housing 120, and FIG. 11 is a cross-sectional view illustrating the handle housing 120 of FIGS. 9 and 10 cut in an A-A' direction.

The handle housing 120 may form an external shape of the applicator 10 together with the body housing 110. The handle housing 120 may be gripped or pressed by a user in an insertion process of the transcutaneous sensor member 330 into subcutaneous tissue. Although FIGS. 9 to 11 illustrate a cup-shaped handle housing 120, the shape of the handle housing 120 is not necessarily limited to the cup shape, and may be variously modified and applied as long as it can achieve the functions described below. However, at one end of the handle housing 120, the cap 50 may be detachably coupled. The cap may be detachably coupled by screw-coupling, and therefore, it may be more preferable that one end of the handle housing 120 in which a screw thread for the screw-coupling is formed is provided to have a cylindrical structure.

In an interior of the handle housing 120, a first interior space 1202 may be formed, and the first interior space 1202 may be connected to the exterior by a first opening 1201 formed at one end of the handle housing 120. A push arm 1230 may be formed to extend along the first direction from an inner one surface of the handle housing 120 facing the first opening 1201.

The push arm 1230 may be provided to interact with a fixing portion 1130 to be described later, and in an assembly process of the applicator 10, by the push arm 1230 pressing and moving the fixing portion 1130, a temporary movement restriction of the sensor unit carrier 130 by the fixing portion 1130 may be released. A push arm slit 1232 may be formed to extend from a leading end portion of the push arm 1230 in a direction opposite to a protruding direction of the push arm 1230 in a shape dividing an end portion of the push arm 1230. An extension portion 1136 provided in the fixing portion 1130 to be described later may be disposed in the push arm slit 1232, so that during an assembly process of the applicator 10 or in an insertion process of the transcutaneous sensor member 330 into subcutaneous tissue, movement interference of the extension portion 1136 with respect to the push arm 1230 may be excluded. Although FIGS. 9 to 11 illustrate the push arm 1230 formed symmetrically with respect to the push arm slit 1232, the shape of the push arm 1230 is not necessarily limited thereto, and as long as it is a structure capable of pressing and moving the fixing portion 1130 in the assembly process of the applicator 10, it may be variously modified and applied. The push arm 1230 may be means for pressing and moving the fixing portion 1130 in the assembly process of the applicator 10.

On an inner one surface of the handle housing 120 facing the first opening 1201, a carrier fixing fence 1207 disposed at a position adjacent to the push arm 1230 may be provided. The carrier fixing fence 1207 may be disposed upright in a shape corresponding to an entire or partial circumferential portion at one end portion side of the sensor unit carrier 130. After assembly of the applicator 10, the sensor unit carrier 130 may be disposed at a position in which at least one surface of the sensor unit carrier 130 is adjacent to an inner surface of the carrier fixing fence 1207 or in close contact with the inner surface of the carrier fixing fence 1207. **In** this case, the sensor unit carrier 130 may be disposed at a position in which one end portion of the sensor unit carrier 130 is spaced apart at a predetermined interval from an inner one surface of the handle housing 120 facing the first opening 1201, or at a position in which one end portion of the sensor unit carrier 130 is in contact with the inner one surface of the handle housing 120 facing the first opening 1201.

When a user presses the handle housing 120 in the first direction for insertion of the transcutaneous sensor member 330 into subcutaneous tissue, the sensor unit carrier 130 may be switched to a state in which one end portion of the sensor unit carrier 130 is in contact with the inner one surface of the handle housing 120 facing the first opening 1201, or may maintain a state in which it is in contact with the inner one surface of the handle housing 120 facing the first opening 1201, and may move together with the handle housing 120 along the first direction. **In** this case, the sensor unit carrier 130 may move in the first direction while maintaining a state in which at least one surface of the sensor unit carrier 130 is firmly in close contact with the inner one surface of the carrier fixing fence 1207, and after completion of insertion of the transcutaneous sensor member 330 into the subcutaneous tissue, it is possible to effectively prevent the handle housing 120 from returning to an initial position.

Meanwhile, the sensor unit carrier 130 may move together with the handle housing 120 in the first direction while maintaining a state spaced apart at a predetermined interval from the inner one surface of the handle housing 120 facing the first opening 1201, and during an insertion process of the transcutaneous sensor member 330 into the subcutaneous tissue or after completion of the insertion of the transcutaneous sensor member 330 into the subcutaneous tissue, one end portion of the sensor unit carrier 130 may be switched to a state in which it is in contact with the inner one surface of the handle housing 120 facing the first opening 1201. **In** this case, at least one surface of the sensor unit carrier 130 may be more firmly in close contact with the inner one surface of the carrier fixing fence 1207 during the insertion process of the transcutaneous sensor member 330 into the subcutaneous tissue or after completion of the insertion of the transcutaneous sensor member 330 into the subcutaneous tissue, and as one end of the sensor unit carrier 130 engages with the carrier fixing fence 1207, it is possible to effectively prevent the handle housing 120 from returning to the initial position.

On an inner side surface of the handle housing 120 forming the first interior space 1202, a guide protrusion 1210 extending along the first direction in a shape protruding toward the first interior space 1202 may be provided. A leading end portion of the guide protrusion 1210 may be disposed in an interior of a handle housing guide groove 1142 formed to be recessed on an outer surface of a body housing 110 to be described later, and through interaction between the guide protrusion 1210 and the handle housing guide groove 1142, a movement direction of the handle housing 120 moving in the first direction may be guided. The guide protrusion 1210 may be provided in a pair in a symmetrical shape on the inner side surface of the handle housing 120, and the handle housing guide groove 1142 may also be formed to be recessed at a position corresponding to the guide protrusion 1210 in a shape corresponding to the guide protrusion 1210. Meanwhile, although a case in which the guide protrusion 1210 is formed on the handle housing 120 and the handle housing guide groove 1142 is formed on the body housing 110 has been exemplarily described, cases in which a guide groove is formed on the handle housing and a guide protrusion is formed on the body housing may be included.

A first movement restriction portion 1220 may be formed to protrude toward a central portion of the first interior space 1202 from the inner side surface of the handle housing 120 forming the first interior space 1202 of the handle housing 120. The first movement restriction portion 1220 may interact with a second movement restriction portion 1150 provided in the body housing 110 to be described later. By the interaction between the first movement restriction portion 1220 and the second movement restriction portion 1150, it is possible to restrict the handle housing 120 coupled to the body housing 110 from being arbitrarily deviated from the body housing 110, or to restrict the handle housing 120 from moving in a direction opposite to the first direction after insertion of the transcutaneous sensor member 330 into subcutaneous tissue. The first movement restriction portion 1220 may be provided as a latch structure having a wedge-shaped cross-section provided with an inclined surface 1221 and a support surface 1222, but the shape of the first movement restriction portion 1220 is not necessarily limited thereto, and as long as it is a shape capable of restricting the handle housing 120 from being arbitrarily deviated from the body housing 110, or restricting the handle housing 120 from moving in a direction opposite to the first direction after insertion of the transcutaneous sensor member 330 into the subcutaneous tissue through interaction with the second movement restriction portion 1150, it may be applied without limitation. The first movement restriction portion 1220 may be means for restricting the handle housing 120 from being arbitrarily deviated from the body housing 110, or means for restricting the handle housing 120 from moving in a direction opposite to the first direction after insertion of the transcutaneous sensor member 330 into the subcutaneous tissue through interaction with the second movement restriction portion 1150.

On an outer surface of one end portion side of the handle housing 120 where the first opening 1201 is formed, a screw threaded portion 1240 for screw-coupling with the cap 50 may be provided. The screw threaded portion 1240 formed in the handle housing 120 and a screw threaded portion 540 formed in the cap 50 to be described later may be screw-coupled so that the handle housing 120 and the cap 50 may be coupled to each other in a detachable manner. On an outer surface of the handle housing 120, a locking protrusion portion 1242 protruding toward the outside from the outer surface of the handle housing 120 and extending along a circumferential direction of the handle housing 120 may be provided. Since the locking protrusion portion 1242 may be provided in a structure capable of contacting a leading end portion of the cap 50, during screw-coupling between the handle housing 120 and the cap 50, it is possible to prevent excessive tightening between the screw threaded portion 1240 of the handle housing 120 and the screw threaded portion 540 of the cap 50. Meanwhile, a structure may be provided in which, in a state where the handle housing 120 and the cap 50 are mutually coupled, the locking protrusion portion 1242 and an end portion of the cap 50 are in close contact, and in this case, it is possible to effectively prevent external contaminants or moisture from being introduced into an interior of the applicator assembly 1.

### Body housing

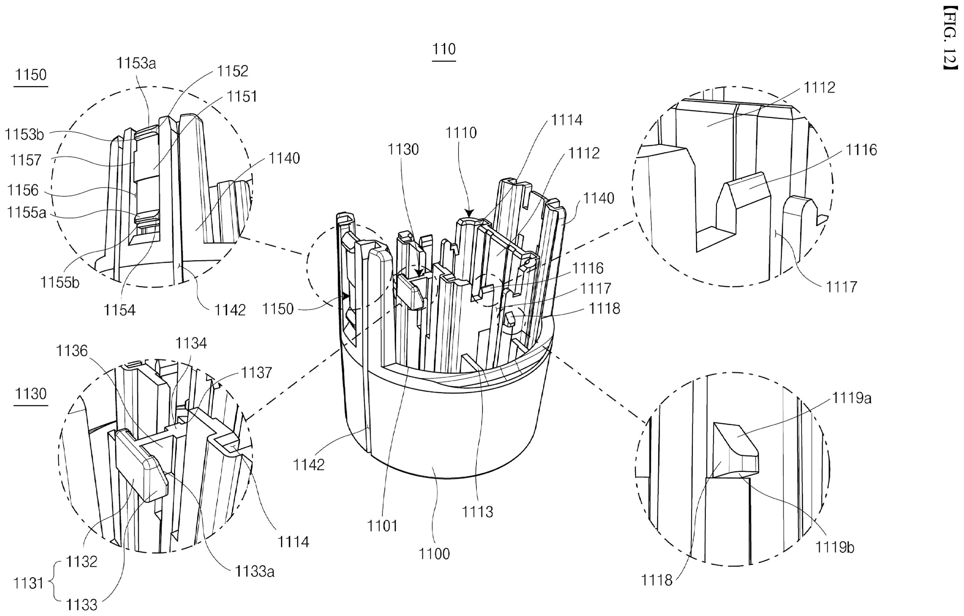

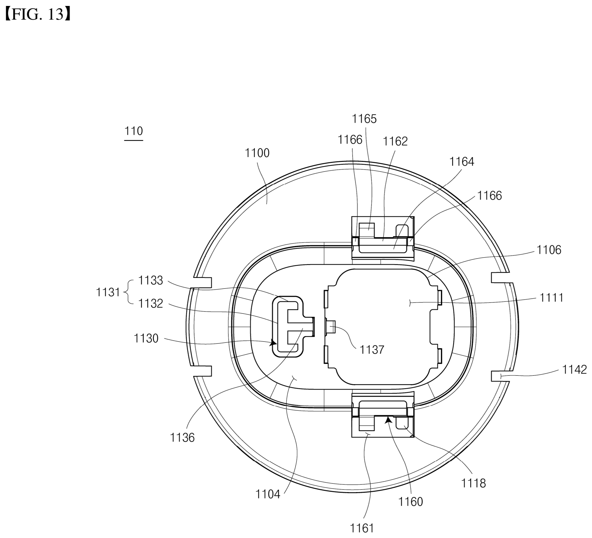

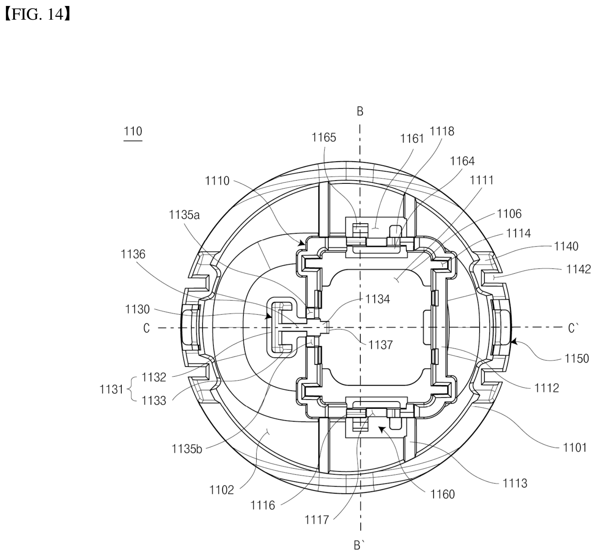

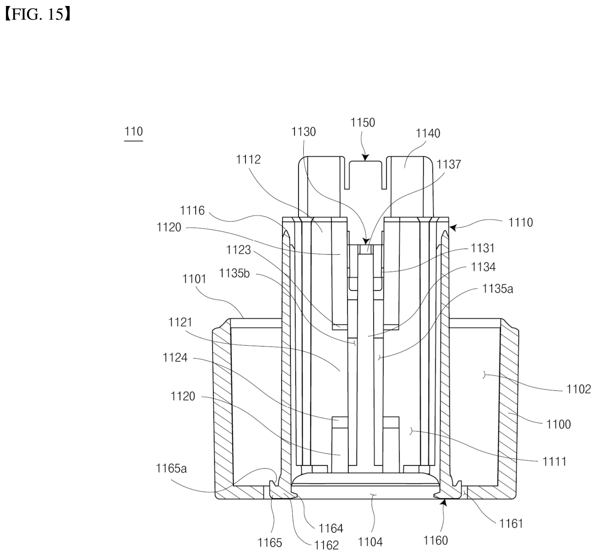

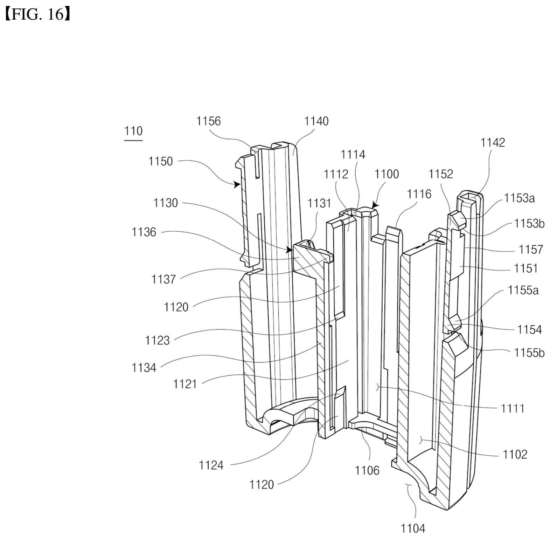

FIG. 12 is a perspective view and a partially enlarged view illustrating an exemplary embodiment of the body housing 110, FIG. 13 is a bottom view illustrating an exemplary embodiment of the body housing 110, and FIG. 14 is a plan view illustrating an exemplary embodiment of the body housing 110. FIG. 15 is a cross-sectional view of the body housing 110 cut along B-B' of FIG. 14, and FIG. 16 is a partial cross-sectional perspective view of the body housing 110 cut along C-C' of FIG. 14.

The body housing 110 may be configured to support the sensor unit carrier 130 disposed in an interior of the applicator 10 to guide a movement direction of the sensor unit carrier 130 and to restrict a movement range of the sensor unit carrier 130, and at one end of the body housing 110, the transmission unit 40 for attachment to the human body B may be separably and fixedly disposed.

The body housing 110 may include a body housing body portion 1100 having a circumferential surface of a shape corresponding to the first interior space 1202 of the handle housing 120. In an interior of the body housing body portion 1100, a second interior space 1102 may be provided, and the second interior space 1102 may be connected to the exterior through a second opening 1101 formed at one end of the body housing 110 adjacent to the handle housing 120. When the body housing 110 and the handle housing 120 are assembled to be coupled to each other, the second interior space 1102 formed in the body housing 110 and the first interior space 1202 formed in the handle housing 120 may be connected to each other to form an interior space (not illustrated) of the interior of the applicator 10 separated from the exterior. Meanwhile, the other end side of the body housing 110 facing the one end where the second opening 1101 is formed may be provided in a closed form.

**In** the interior of the body housing 110, a column 1110 in which a first movement space 1111 is formed to penetrate along the first direction may be disposed upright. The column 1110 may include a plurality of partition walls 1112 extending along a direction opposite to the first direction from an inner surface of the closed other end side of the body housing 110 and disposed upright. The plurality of partition walls 1112 may be provided to surround the first movement space 1111 from a side surface side, and by the plurality of partition walls 1112, the second interior space 1102 and the first movement space 1111 may be separated. A sensor unit carrier body 1310 of the sensor unit carrier 130 to be described later, the needle carrier 140, and the sensor unit 30 may move toward the transmission unit 40 through the first movement space 1111 in an insertion process of the transcutaneous sensor member 330 into subcutaneous tissue.

Meanwhile, although a hexagonal column form of the column 1110 will be described as an example hereinafter, a shape of the column 1110 of the present invention is not limited thereto, and as long as it is a shape capable of providing a movement path of the sensor unit carrier body 1310, the needle carrier 140, and the sensor unit 30 in the insertion process of the transcutaneous sensor member 330 into the subcutaneous tissue, it may be variously modified and applied.

On an outer surface of one or more of the plurality of partition walls 1112, a first acceleration latch 1118 provided in a shape protruding toward the outside from the outer surface of the partition wall 1112 may be provided. The first acceleration latch 1118 may interact with a second acceleration latch 1328 provided in the sensor unit carrier 130 to be described later so as to grant a movement initiation condition of the sensor unit carrier 130 to move in the first direction only when a force of a predetermined amount or more is applied to the sensor unit carrier 130. That is, since insertion of the transcutaneous sensor member 330 into subcutaneous tissue is carried out only when a force sufficient to release a movement constraint of the second acceleration latch 1328 by the first acceleration latch 1118 is applied to the handle housing 120, a situation in which the transcutaneous sensor member 330 is arbitrarily fired in an unintended situation by a user may be effectively avoided. Meanwhile, in order for the transcutaneous sensor member 330 to be normally inserted into the subcutaneous tissue, the needle body 1402 is required to move at a speed equal to or greater than a reference speed so that a leading end of the needle body 1402 may penetrate a skin surface, and through interaction between the first acceleration latch 1118 and the second acceleration latch 1328, acceleration conditions may be granted so as to fire the needle body 1402 at a speed equal to or greater than the reference speed, thereby effectively inducing normal insertion of the transcutaneous sensor member 330 into the subcutaneous tissue. The first acceleration latch 1118 may be provided as a latch structure having a wedge-shaped cross-section provided with an inclined surface 1119a and a support surface 1119b, but a shape of the first acceleration latch 1118 is not necessarily limited thereto, and as long as the shape is capable of granting a movement initiation condition or an acceleration condition of the sensor unit carrier 130 through interaction with the second acceleration latch 1328, it may be variously modified and applied. The first acceleration latch 1118 may be means for granting a movement initiation condition or an acceleration condition of the sensor unit carrier 130 through interaction with the second acceleration latch 1328.

At a leading end portion of the partition wall 1112 provided with the first acceleration latch 1118, a bridge pressing portion 1116 having a shape in which a cross-section decreases toward a leading end may be provided. The bridge pressing portion 1116 may be provided to press a bridge 1330 provided in the sensor unit carrier 130 to be described later so as to deform or cut the bridge 1330. The bridge pressing portion 1116 may be disposed to be spaced apart at a predetermined interval at a position facing the bridge 1330. As the sensor unit carrier 130 moves in the first direction, a distance between the bridge 1330 and the bridge pressing portion 1116 may become closer. That is, in an initial step of movement of the sensor unit carrier 130 in the first direction, the bridge 1330 provided in the sensor unit carrier 130 is moved to a position in close contact with the bridge pressing portion 1116, and only when a force in the first direction sufficient to cut or deform the bridge 1330 pressed by the bridge pressing portion 1116 is applied to the sensor unit carrier 130, movement of the sensor unit carrier 130 in the first direction may be completed. When the bridge 1330 is not cut or deformed despite the movement of the sensor unit carrier 130 in the first direction, a state in which the bridge 1330 is caught by the bridge pressing portion 1116 is maintained, and the sensor unit carrier 130 may be placed in a situation where the sensor unit carrier 130 can no longer move in the first direction. That is, not only may arbitrary firing of the transcutaneous sensor member 330 in an unintended situation by a user be effectively prevented through interaction between the bridge 1330 and the bridge pressing portion 1116, but also sufficient acceleration conditions for normal insertion of the transcutaneous sensor member 330 into the subcutaneous tissue may be granted.

At the partition wall 1112 provided with the first acceleration latch 1118, a carrier slit 1117 formed to penetrate in a shape incising the partition wall 1112 along a direction parallel to a direction opposite to the first direction from the leading end portion of the partition wall 1112 may be provided. The carrier slit 1117 may more preferably be formed to penetrate between the first acceleration latch 1118 and the bridge pressing portion 1116 in terms of structural simplification. The carrier slit 1117 and the first movement space 1111 may be in communication, and by the carrier slit 1117, the first movement space 1111 and the second interior space 1102 may be in communication. In a process in which the sensor unit carrier 130 moves in a first direction, an extension arm connection portion 1322 of the sensor unit carrier 130 to be described later may be introduced into an interior of the carrier slit 1117. As the extension arm connection portion 1322 is introduced into the interior of the carrier slit 1117, interference with movement in the first direction of the extension arm connection portion 1322 by the partition wall 1112 may be excluded, and movement of the sensor unit carrier 130 may be guided by the carrier slit 1117. As the extension arm connection portion 1322 moves along the carrier slit 1117, the sensor unit 30 separably fixed to an end portion of the sensor unit carrier 130 may be accurately delivered to the seating groove 412 of the transmission unit 40.

At least one of the first acceleration latch 1118, the bridge pressing portion 1116, and the carrier slit 1117 may be provided in pairs, and may be respectively disposed symmetrically on partition walls 1112 facing each other.

On an inner surface of the partition wall 1112 forming the first movement space 1111, a carrier guide groove 1114 may be formed to be recessed in a direction intersecting the first direction, and the carrier guide groove 1114 may be formed to extend along the first direction. In one example, the carrier guide groove 1114 may be formed to be recessed in a shape directed from the inner surface of the partition wall 1112 toward the outside.

The sensor unit carrier 130 may be provided with a carrier guide protrusion 1310a having a shape corresponding to the carrier guide groove 1114. The carrier guide protrusion 1310a may protrude from one end of the sensor unit carrier body 1310 in a direction intersecting the first direction and may be formed in a shape extending along the first direction. At least one end of the carrier guide protrusion 1310a may be disposed in the interior of the carrier guide groove 1114. A leading end portion of the carrier guide protrusion 1310a may be disposed in the carrier guide groove 1114 to guide movement of the sensor unit carrier 130 so that the sensor unit carrier 130 moves along the first direction. In the drawings, four carrier guide protrusions 1310a disposed at respective corner portions of the sensor unit carrier 130 and the carrier guide grooves 1114 formed on an inner surface of the column 1110 in positions and in a number corresponding thereto are exemplarily illustrated. However, a shape and number of the carrier guide protrusion 1310a and the carrier guide groove 1114 are not necessarily limited to matters illustrated in the drawings, and as long as the shape and number are capable of guiding movement of the sensor unit carrier 130 in the first direction, they may be variously modified and applied. The carrier guide protrusion 1310a and the carrier guide groove 1114 may be means for guiding movement of the sensor unit carrier 130 in the first direction.

In the interior of the second interior space 1102, a partition wall support portion 1113 provided to support the column 1110 may be disposed upright. The partition wall support portion 1113 may be disposed so that one end thereof is connected to the partition wall 1112 and the other end thereof is connected to an inner side wall of the body housing body portion 1100, thereby improving structural stability and rigidity of the column 1110 and the body housing body portion 1100.

A body extension portion 1140 may be provided in a pair at one end portion of the body housing body portion 1100 in a shape extending along a direction opposite to the first direction from the one end portion of the body housing body portion 1100 where the second opening 1101 is formed. The handle housing guide groove 1142 may be formed in a shape extending along the first direction from an outer surface of a leading end portion side of a body extension portion 1140 to an outer surface of the other end portion side of the body housing body portion 1100. That is, the handle housing guide groove 1142 may be formed to be recessed so as to extend from the outer surface of the body extension portion 1140 to the outer surface of the body housing body portion 1100 in a shape terminating the body extension portion 1140 and the body housing body portion 1100. A leading end portion of the guide protrusion 1210 of the handle housing 120 may be disposed in an interior of the handle housing guide groove 1142 so that a movement direction of the handle housing 120 moving along the first direction may be guided.

On a leading end portion side of the body extension portion 1140, the second movement restriction portion 1150 may be provided. On the leading end portion of the body extension portion 1140, a movement restriction latch accommodation portion 1156 having a shape incising the body extension portion 1140 along the first direction from the leading end of the body extension portion 1140 may be formed to penetrate. A movement restriction body 1151 may be disposed to be accommodated in the movement restriction latch accommodation portion 1156, and both side end portions on a central portion side of the movement restriction body 1151 may be connected to the body extension portion 1140 by a body connection portion 1157. Since both side end portions on the central portion side of the movement restriction body 1151 are connected to the body extension portion 1140 through the body connection portion 1157, when an external force is applied to the movement restriction body 1151, the movement restriction body 1151 may be deformed in a state of being accommodated in the movement restriction latch accommodation portion 1156. In the present invention, the term "deformed" is interchangeably used with "bent", "curved", "twisted" (including torsionally deformed) or "distorted". The deformation of the movement restriction body 1511 is temporary and reversible, and when the external force is removed, it returns to its original position and/or shape. On an outer surface of a one side end portion of the movement restriction body 1151, a first movement restriction latch 1152 may be provided, and on an outer surface of the other side end portion of the movement restriction body 1151, a second movement restriction latch 1154 may be provided. The first movement restriction latch 1152 and the second movement restriction latch 1154 may preferably be disposed to be spaced apart from each other along the first direction.

The first movement restriction latch 1152 may be provided as a latch structure having a wedge-shaped cross-section provided with a first inclined surface 1153a and a first support surface 1153b, and likewise, the second movement restriction latch 1154 may be provided as a latch structure having a wedge-shaped cross-section provided with a second inclined surface 1155a and a second support surface 1155b. The first movement restriction latch 1152 and the second movement restriction latch 1154 may interact with the first movement restriction portion 1220 of the above-described handle housing 120 to constrain movement of the handle housing 120.

The support surface 1222 of the first movement restriction portion 1220 may be formed along a direction substantially parallel to the second direction, and the inclined surface 1221 of the first movement restriction portion 1220 may be inclinedly disposed so as to become adjacent to an inner side wall of the handle housing 120 along the first direction from an end portion of the protruding support surface 1222. The first support surface 1153b of the first movement restriction latch 1152 may be formed along a direction substantially parallel to the second direction, and the first inclined surface 1153a of the first movement restriction latch 1152 may be inclinedly disposed so as to become adjacent to one end portion side of the movement restriction body 1151 along a direction opposite to the first direction from an end portion of the protruding first support surface 1153b. The second support surface 1155b of the second movement restriction latch 1154 may be formed along a direction substantially parallel to the second direction, and the second inclined surface 1155a of the second movement restriction latch 1154 may be inclinedly disposed so as to become adjacent to the other end portion side of the movement restriction body 1151 along a direction opposite to the first direction from an end portion of the protruding second support surface 1155b.

When the handle housing 120 and the body housing 110 are assembled, in a process in which the handle housing 120 relatively moves with respect to the body housing 110 in the first direction, the inclined surface 1221 of the first movement restriction portion 1220 may reach a state of coming into contact with the first inclined surface 1153a of the first movement restriction latch 1152. Thereafter, as the handle housing 120 further moves along the first direction, one end of a movement restriction body 1151 may be deformed toward the second interior space 1102, and accordingly, the first movement restriction portion 1220 may pass through the first movement restriction latch 1152. After the first movement restriction portion 1220 passes through the first movement restriction latch 1152, the movement restriction body 1151 may be restored to a state before deformation. After the first movement restriction portion 1220 passes through the first movement restriction latch 1152 and coupling of the handle housing 120 and the body housing 110 is completed, since the support surface 1222 of the first movement restriction portion 1220 and the first support surface 1153b of the first movement restriction latch 1152 are placed in a state of facing each other, even if a force in a direction opposite to the first direction is applied to the handle housing 120, the support surface 1222 of the first movement restriction portion 1220 and the first support surface 1153b of the first movement restriction latch 1152 may support each other, thereby preventing the handle housing 120 from being arbitrarily deviated from the body housing 110.

In an insertion process of the transcutaneous sensor member 330 into the subcutaneous tissue, in a state in which the inclined surface 1221 of the first movement restriction portion 1220 comes into contact with the second inclined surface 1155a of the second movement restriction latch 1154, the handle housing 120 may move in the first direction, and the other end of the movement restriction body 1151 may be deformed toward the second interior space 1102 so that the first movement restriction portion 1220 passes through the second movement restriction latch 1154. After the first movement restriction portion 1220 passes through the second movement restriction latch 1154, the movement restriction body 1151 may be restored to a state before deformation. After the first movement restriction portion 1220 passes through the second movement restriction latch 1154 and the insertion of the transcutaneous sensor member 330 into the subcutaneous tissue is completed, since the support surface 1222 of the first movement restriction portion 1220 and the second support surface 1155b of the second movement restriction latch 1154 are placed in a state of facing each other, movement of the handle housing 120 in the direction opposite to the first direction may be restricted. That is, after the insertion of the transcutaneous sensor member 330 into the subcutaneous tissue, through interaction between the first movement restriction portion 1220 and the second movement restriction portion 1150, restoration of the handle housing 120 to a position before insertion of the transcutaneous sensor member 330 may be restricted, and accordingly, reuse of the applicator 10 after firing may be effectively prevented.

In one of the plurality of partition walls 1112, the fixing portion 1130 for restricting arbitrary movement of the sensor unit carrier 130 during assembly of the applicator 10 may be provided. In a leading end portion of the partition wall 1112 provided with the fixing portion 1130, a first incision groove 1135a and a second incision groove 1135b formed to incise the partition wall 1112 along the first direction from a leading end of the partition wall 1112 may be formed to penetrate in parallel while being spaced apart from each other. Between the first incision groove 1135a and the second incision groove 1135b, a support 1134 may be disposed upright in a direction parallel to the first direction, and on one surface of the support 1134 facing the first movement space 1111, a fixing protrusion portion 1137 having a shape protruding toward the first movement space 1111 may be provided. On the other surface of the support 1134 opposite to the one surface of the support 1134 on which the fixing protrusion portion 1137 is formed, a pressing movement extension portion 1136 extending from the other surface of the support 1134 may be provided, and on a protruding leading end portion of the pressing movement extension portion 1136, a pressing movement portion 1131 may be provided. The pressing movement portion 1131 may include a pressing movement body portion 1132 connected to the pressing movement extension portion 1136, and pressing movement wing portions 1133 provided at both side ends of the pressing movement body portion 1132. The pressing movement wing portion 1133 may be provided with a pressing movement inclined surface 1133a having a shape coming close to the partition wall 1112 along the first direction.

As described below, on one surface of the sensor unit carrier body 1310, a fixing groove 1340 having a shape corresponding to the fixing protrusion portion 1137 may be formed to be recessed. When defining a position of the sensor unit carrier 130 in a state where the handle housing 120 is not pressed by a user (that is, a state where firing of the transcutaneous sensor member 330 and insertion into subcutaneous tissue are not performed) as an initial position, and defining a position of the sensor unit carrier 130 in a state after the handle housing 120 is pressed by the user (that is, a state after firing of the transcutaneous sensor member 330 and insertion into subcutaneous tissue are performed) as an insertion position, it may be preferable that the fixing groove 1340 is formed to be recessed on one surface of the sensor unit carrier body 1310 so that the fixing groove 1340 of the sensor unit carrier 130 positioned at the initial position and the fixing protrusion portion 1137 are positioned at a position corresponding to each other.

In a process of fitting the sensor unit carrier body 1310 into the first movement space 1111 in order to assemble the applicator 10, the fixing protrusion portion 1137 comes into contact with one surface of the sensor unit carrier body 1310, whereby the support 1134 is deformed outward, and the fixing portion 1130 is maintained in a state of being moved in a direction away from the first movement space 1111. Subsequently, when the sensor unit carrier body 1310 is pushed into the first movement space 1111 along the first direction until the fixing protrusion portion 1137 and the fixing groove 1340 correspond to each other in position, a leading end portion of the fixing protrusion portion 1137 is introduced into and disposed in an interior of the fixing groove 1340, and the support 1134 is restored to a state before deformation. As the leading end portion of the fixing protrusion portion 1137 is introduced into the interior of the fixing groove 1340, arbitrary movement of the sensor unit carrier 130 in the first direction or in a direction opposite to the first direction is restricted, and subsequent coupling work of the handle housing 120 and the body housing 110 may be performed.

In a process of coupling the handle housing 120 to the body housing 110, a leading end portion of the push arm 1230 provided in the handle housing 120 comes into contact with the pressing movement inclined surface 1133a of the pressing movement wing portion 1133, and the leading end portion of the push arm 1230 moves in the first direction in a state of being in contact with the pressing movement inclined surface 1133a, to press the pressing movement wing portion 1133, whereby the support 1134 is deformed outward and the pressing movement portion 1131 may be pushed in a direction away from the sensor unit carrier body 1310. As the pressing movement portion 1131 is pushed in a direction away from the sensor unit carrier body 1310, the leading end portion of the fixing protrusion portion 1137 moves to a position deviated from the fixing groove 1340, and accordingly, the arbitrary movement restriction of the sensor unit carrier 130 by the fixing portion 1130 may be released. Since assembly of the applicator 10 may be performed in a state where the sensor unit carrier 130 maintains the initial position by the fixing portion 1130, work efficiency during the assembly work of the applicator 10 may be more effectively improved.

On a closed one end side of the body housing 110 facing the second opening 1101, a transmission unit accommodation portion 1104 capable of accommodating the transmission unit 40 may be provided. The transmission unit accommodation portion 1104 may be formed in a shape recessed from the exterior of the body housing 110 toward the second interior space 1102. The transmission unit accommodation portion 1104 may preferably be formed to be recessed in a shape corresponding to the transmission unit housing 410 so as to be able to accommodate the transmission unit housing 410 in its interior. In a state where the transmission unit 40 is accommodated in the transmission unit accommodation portion 1104, when one end of the applicator 10 is brought into close contact with skin, the transmission unit accommodation portion 1104 may be formed to be recessed to a recessed depth such that the adhesive member 430 provided in the transmission unit 40 may be attached to the skin.

At one end of the body housing 110 where the transmission unit accommodation portion 1104 is formed, a third opening 1106 connecting the transmission unit accommodation portion 1104 and the first movement space 1111 may be formed to penetrate. In an insertion process of the transcutaneous sensor member 330 into the subcutaneous tissue, the sensor unit 30 that has moved along the first direction from the first movement space 1111 may pass through the third opening 1106 and be delivered to the seating groove 412 of the transmission unit 40 accommodated in the transmission unit accommodation portion 1104.

At both side ends of the transmission unit accommodation portion 1104, transmission unit support portions 1160 for preventing arbitrary deviation of the transmission unit 40 from the transmission unit accommodation portion 1104 may be provided. At both side ends of the transmission unit accommodation portion 1104, support hook accommodation spaces 1161 formed to penetrate a closed one end of the body housing 110 may be provided. A transmission unit support hook 1162 configured to be capable of supporting the transmission unit 40 may be disposed to be accommodated in the support hook accommodation space 1161. The transmission unit support hook 1162 may be supported by support hook connection portions 1166, which extend respectively from an end portion of the body housing 110 forming the support hook accommodation space 1161 and are connected to both side ends of the transmission unit support hook 1162. Since the transmission unit support hook 1162 is connected to the support hook connection portion 1166 extending from the end portion of the body housing 110 and disposed in the support hook accommodation space 1161, when an external force is applied to the transmission unit support hook 1162, the support hook connection portion 1166 may be deformed so that the position of the transmission unit support hook 1162 may be changed.

The transmission unit support hook 1162 may include a catching portion 1164 formed to protrude in a direction toward the transmission unit accommodation portion 1104 and a pushed portion 1165 formed to protrude in a direction opposite to the transmission unit accommodation portion 1104. A leading end portion of the catching portion 1164 may be inserted into the transmission unit housing groove 417 of the transmission unit 40 accommodated in the transmission unit accommodation portion 1104, and the transmission unit 40 accommodated in the transmission unit accommodation portion 1104 may be supported by the catching portion 1164 having its leading end portion inserted into the transmission unit housing groove 417, thereby preventing arbitrary deviation from the transmission unit accommodation portion 1104. The pushed portion 1165 may be formed with a pushed portion inclined surface 1165a inclinedly disposed in a shape becoming closer to the transmission unit accommodation portion 1104 along the first direction. By pressing the pushed portion inclined surface 1165a with an extension arm pushing portion 1326 of the sensor unit carrier 130 to be described later, the transmission unit support hook 1162 may be moved in a direction away from the transmission unit accommodation portion 1104. That is, in the insertion process of the transcutaneous sensor member 330 into the subcutaneous tissue, the sensor unit carrier 130 moves together with the sensor unit 30 along the first direction, and at the same time when the sensor unit 30 is delivered from the sensor unit carrier 130 to the transmission unit 40, or at a point in time somewhat earlier than a point in time when the sensor unit 30 is delivered from the sensor unit carrier 130 to the transmission unit 40, the extension arm pushing portion 1326 may press the pushed portion inclined surface 1165a so that movement restriction of the transmission unit 40 by the catching portion 1164 may be released.

Meanwhile, in a process of disposing the transmission unit 40 in the transmission unit accommodation portion 1104 in manufacturing of the applicator assembly 1, the transmission unit support hook 1162 may be pressed by the transmission unit housing 410 to be moved in a direction away from the transmission unit accommodation portion 1104, and when the transmission unit 40 is fully seated in the transmission unit accommodation portion 1104, the moved transmission unit support hook 1162 may be restored to its original state so that a leading end portion of the catching portion 1164 may be disposed in an interior of the transmission unit housing groove 417. The transmission unit support portion 1160 is provided so as to release movement constraint of the transmission unit 40 only when intended by a worker or a user in an assembly process or a using process of the applicator assembly 1, and thus, assembling work convenience and convenience of use of the applicator assembly 1 may be more effectively improved.

On one or more inner surfaces of the partition wall 1112 forming the first movement space 1111, a grip arm guide groove 1120 extending along the first direction may be formed in a shape recessed toward the outside. A grip arm 1422 of the needle carrier 140 to be described later may be disposed in the grip arm guide groove 1120 so that a movement direction of the needle carrier 140 may be guided. At a central portion of the grip arm guide groove 1120, a step portion 1121 protruding to a height substantially corresponding to one surface of the partition wall 1112 in which the grip arm guide groove 1120 is formed may be formed, and at one end and the other end of the step portion 1121, a first step inclined surface 1123 and a second step inclined surface 1124 may respectively be formed. The first step inclined surface 1123 may be inclinedly disposed in a shape becoming adjacent to the first movement space 1111 along the first direction, and the second step inclined surface 1124 may be inclinedly disposed in a shape away from the first movement space 1111 along the first direction.

During assembly of the applicator assembly 1, the needle carrier 140 may be introduced into an interior of the first movement space 1111 along the first direction, and in a state where the grip arm 1422 is disposed in the grip arm guide groove 1120, the needle carrier 140 may move to an initial position. When the needle carrier 140 is disposed in the initial position, a grip arm protrusion portion 1424 formed on the grip arm 1422 may pass through the first step inclined surface 1123 or be positioned at a position immediately before passing through the first step inclined surface 1123. Subsequently, as the needle carrier 140 further moves along the first direction, the grip arm protrusion portion 1424 may come into close contact with the step portion 1121 and pressed inward, and in a process in which the needle carrier 140 moves, the sensor unit 30 may be maintained in a more firmly fixed state on the inwardly pressed grip arm 1422.

Meanwhile, in a process in which the needle carrier 140 further moves along the first direction to move to an insertion position, the grip arm protrusion portion 1424 may pass through the second step inclined surface 1124, and as the grip arm protrusion portion 1424 passes through the second step inclined surface 1124, pressing of the grip arm protrusion portion 1424 by the step portion 1121 may be released. That is, in the insertion process of the transcutaneous sensor member 330 into the subcutaneous tissue, the pressing of the grip arm protrusion portion 1424 by the step portion 1121 may be released simultaneously with delivering the sensor unit 30 to the seating groove 412 of the transmission unit 40 or before delivering the sensor unit 30 to the seating groove 412 of the transmission unit 40. It is possible not only to effectively prevent the sensor unit 30 from being deviated from a designated position in an interior of the applicator 10 during the insertion process of the transcutaneous sensor member 330 into the subcutaneous tissue, but also to effectively prevent a phenomenon in which the sensor unit 30 is brought along in a direction opposite to the first direction by the grip arm 1422 immediately after the insertion of the transcutaneous sensor member 330 into the subcutaneous tissue.

### Sensor unit carrier

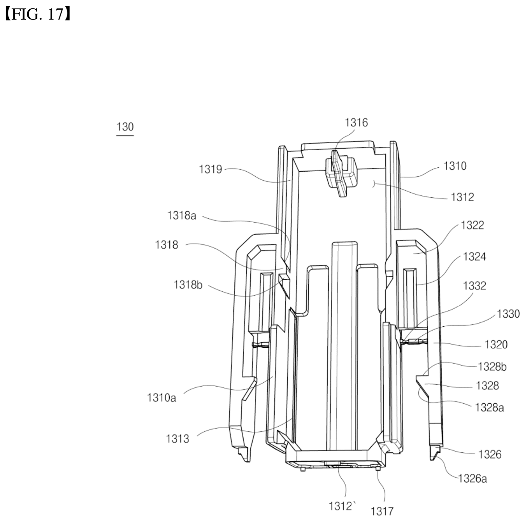

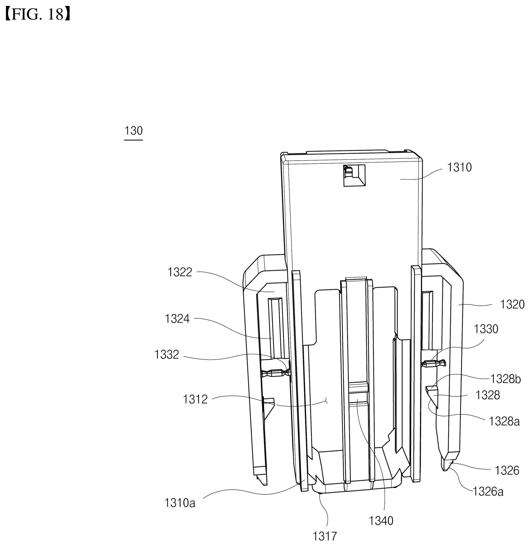

FIG. 17 and FIG. 18 are perspective views illustrating an exemplary embodiment of the sensor unit carrier 130.

The sensor unit carrier 130 may be disposed in an interior of the applicator assembly 1 and is configured to move together with the needle carrier 140 and the sensor unit 30 in the first direction in the insertion process of the transcutaneous sensor member 330 into the subcutaneous tissue. After the insertion of the transcutaneous sensor member 330 into the subcutaneous tissue, the needle carrier 140 may move to a retracted position such that the needle body 1402 is discharged from the skin, whereas the sensor unit carrier 130 may be maintained at an insertion position.

The sensor unit carrier 130 includes the sensor unit carrier body 1310 constituting a skeleton of the sensor unit carrier 130, and the sensor unit carrier body 1310 may be provided with a second movement space 1312 having a front side opened. The needle carrier 140 may move to the retracted position through the second movement space 1312 after the insertion of the transcutaneous sensor member 330 into the subcutaneous tissue. At one end portion of the sensor unit carrier body 1310 adjacent to the third opening 1106 formed in the body housing 110, a fourth opening 1312' connecting the exterior and the second movement space 1312 may be formed to penetrate. The needle body 1402 may protrude to the outside of the sensor unit carrier body 1310 through the fourth opening 1312'. On one end surface of the sensor unit carrier body 1310 facing the third opening 1106, a fixing protrusion 1317 having a shape protruding toward the third opening 1106 may be provided, and the sensor unit 30 may be more firmly fixed to the sensor unit carrier 130 by a leading end portion of the fixing protrusion 1317 inserted into an interior of the fixing groove 317 of the sensor unit 30.

At a corner portion on a side end side of the sensor unit carrier body 1310, the carrier guide protrusion 1310a having a shape protruding toward the outside may be formed to extend along the first direction. A leading end portion of the carrier guide protrusion 1310a may be disposed in an interior of the carrier guide groove 1114 formed in the column 1110, and a movement direction of the sensor unit carrier body 1310 moving along the first direction may be guided by the carrier guide protrusion 1310a and the carrier guide groove 1114. On an inner side surface of the sensor unit carrier body 1310 forming the second movement space 1312, a needle carrier guide protrusion 1313 formed to protrude from the inner side surface of the sensor unit carrier body 1310 toward a central portion of the second movement space 1312 and to extend along the first direction may be provided. A leading end portion of the needle carrier guide protrusion 1313 may be disposed in an interior of a needle guide groove 1415 formed in the needle carrier body 1410 to be described later, and a movement direction of the needle carrier 140 moving in a direction opposite to the first direction may be guided by the needle carrier guide protrusion 1313 and the needle guide groove 1415.

On an inner side surface of the sensor unit carrier body 1310 adjacent to the handle housing 120, a sliding groove 1319 formed to be recessed from an inner surface on a leading end side of the sensor unit carrier body 1310 and to extend along the first direction may be provided. It is preferable that the sliding groove 1319 is formed at a position adjacent to an opened front surface side of the second movement space 1312. A sensor unit carrier detent 1318 having a detent inclined surface 1318a and a detent constraint surface 1318b formed therein may be disposed in the sliding groove 1319. Since a needle carrier latch 1434 of the needle carrier 140 to be described later maintains a state of being constrained to the sensor unit carrier detent 1318, the needle carrier 140 may move together with the sensor unit carrier 130 in the first direction in the insertion process of a transcutaneous sensor member 330 into the subcutaneous tissue. The detent constraint surface 1318b may be formed along a direction substantially parallel to the second direction, and the detent inclined surface 1318a may be inclinedly disposed so as to be adjacent to the sliding groove 1319 along a direction opposite to the first direction from a protruding one end of the detent constraint surface 1318b.

An extension arm 1320 may be provided in a form of protruding toward the exterior from both outer surfaces of the sensor unit carrier body 1310 and extending along the first direction. An extension arm connection portion 1322 may be provided between the sensor unit carrier body 1310 and the extension arm 1320. In the extension arm connection portion 1322, an extension arm guide groove 1324 may be formed to be recessed along the first direction. It may be preferable that the extension arm guide groove 1324 is formed in a position and shape corresponding to the carrier slit 1117 of the column 1110. In a process in which the sensor unit carrier body 1310 moves along the first direction to insert the transcutaneous sensor member 330 into the subcutaneous tissue, the extension arm connection portion 1322 having the extension arm guide groove 1324 formed therein may be introduced into the carrier slit 1117 of the column 1110, and not only a movement direction of the sensor unit carrier 130 moving in the first direction may be guided by the carrier slit 1117 and the extension arm guide groove 1324, but also interference with movement of the sensor unit carrier 130 in the first direction by the column 1110 may be excluded.

On one surface of the extension arm 1320 facing the sensor unit carrier body 1310, the second acceleration latch 1328 formed to protrude toward the sensor unit carrier body 1310 may be provided. The second acceleration latch 1328 may include a support surface 1328b formed along a direction substantially parallel to the second direction, and an inclined surface 1328a inclinedly disposed so as to be gradually adjacent toward the one surface of the extension arm 1320 along the first direction from a protruding end portion of the support surface 1328b. The second acceleration latch 1328 may prevent arbitrary firing of the applicator 10 in an unintended situation by a user through interaction with the first acceleration latch 1118 provided in the body housing 110, may grant sufficient acceleration conditions to the needle body 1402 during the insertion of the transcutaneous sensor member 330 into the subcutaneous tissue, and may prevent the sensor unit carrier 130 from moving in a direction opposite to the first direction after the insertion of the transcutaneous sensor member 330 into the subcutaneous tissue.

When the sensor unit carrier 130 is positioned at an initial position, the inclined surface 1119a of the first acceleration latch 1118 and the inclined surface 1328a of the second acceleration latch 1328 may be maintained in a state spaced apart from each other while facing each other, or in a state in contact with each other. When a user presses the handle housing 120 in the first direction such that the sensor unit carrier 130 moves in the first direction, the inclined surface 1119a of the first acceleration latch 1118 and the inclined surface 1328a of the second acceleration latch 1328 may move while rubbing against each other in a state of being in contact, and in this case, the extension arm 1320 may be deformed toward the outward direction. Subsequently, as the sensor unit carrier 130 moves toward the first direction, contact between the inclined surface 1328a of the second acceleration latch 1328 and the inclined surface 1119a of the first acceleration latch 1118 may be released, and the support surface 1119b of the first acceleration latch 1118 and the support surface 1328b of the second acceleration latch 1328 may be switched to a state facing each other. After the support surface 1119b of the first acceleration latch 1118 and the support surface 1328b of the second acceleration latch 1328 are switched to a state facing each other, the insertion of the transcutaneous sensor member 330 into the subcutaneous tissue may be performed, and after the insertion of the transcutaneous sensor member 330 into the subcutaneous tissue, movement of the sensor unit carrier 130 in a direction opposite to the first direction may be restricted by the facing support surface 1119b of the first acceleration latch 1118 and the support surface 1328b of the second acceleration latch 1328. In the above, although a latch structure having a wedge-shaped cross-section provided with the inclined surfaces 1119a and 1328a and the support surfaces 1119b and 1328b has been exemplarily described in relation to the shape of the first acceleration latch 1118 and the second acceleration latch 1328, the shape of the first acceleration latch 1118 and the second acceleration latch 1328 is not necessarily limited thereto, and may be variously modified and applied as long as the structure can prevent arbitrary firing of the applicator 10 in an unintended situation by a user through interaction, grant sufficient acceleration conditions to the needle body 1402 during the insertion of the transcutaneous sensor member 330 into the subcutaneous tissue, and prevent movement of the sensor unit carrier 130 in the direction opposite to the first direction after the insertion of the transcutaneous sensor member 330 into the subcutaneous tissue. The first acceleration latch 1118 and the second acceleration latch 1328 may be means for prevent arbitrary firing of the applicator 10 in an unintended situation by a user through interaction, means for grant sufficient acceleration conditions to the needle body 1402 during the insertion of the transcutaneous sensor member 330 into the subcutaneous tissue, or means for prevent movement of the sensor unit carrier 130 in the direction opposite to the first direction after the insertion of the transcutaneous sensor member 330 into the subcutaneous tissue.

The bridge 1330 may be disposed between the extension arm 1320 and the sensor unit carrier body 1310. The bridge is configured to restrict movement of the sensor unit carrier from an initial position to an insertion position. The bridge is configured to be cut or deformed when pressed by the bridge pressing portion 1116. One end and the other end of the bridge 1330 may be provided to be connected to one side surface of the sensor unit carrier body 1310 and to one surface of the extension arm 1320 facing the sensor unit carrier body 1310, respectively. Alternatively, only one end of the bridge 1330 may be provided to be connected to either one side surface of the sensor unit carrier body 1310 or one surface of the extension arm 1320 facing the sensor carrier body 1310. It may be preferable that the bridge 1330 is formed at a position facing the bridge pressing portion 1116 of the column 1110, and one or more vulnerable portions 1332 having a relatively small thickness or relatively low rigidity than the other portions of the bridge may be formed in the bridge 1330. The vulnerable portion 1332 may mean a region intended to be cut when the bridge 1330 is pressed by the bridge pressing portion 1116.

At an end portion of the extension arm 1320, the extension arm pushing portion 1326 having an inclined surface 1326a may be formed to protrude toward the first direction. When the sensor unit carrier 130 moves along the first direction and is positioned at an insertion position, the extension arm pushing portion 1326 may press the pushed portion 1165 provided in the transmission unit support hook 1162, and by the pushed portion 1165 being pressed by the extension arm pushing portion 1326, the transmission unit support hook 1162 may be deformed toward the outside away from the transmission unit 40, thereby releasing movement restriction of the transmission unit 40 by the catching portion 1164. The inclined surface 1326a formed in the extension arm pushing portion 1326 and the inclined surface 1165a formed in the pushed portion 1165 are not particularly limited as long as the shape allows the transmission unit support hook 1162 to be deformable toward the outside away from the transmission unit 40 when the pushed portion 1165 is pressed by the extension arm pushing portion 1326. It may be preferable that the extension arm pushing portion 1326 is formed at a position capable of pressing the pushed portion 1165 simultaneously with the sensor unit carrier 130 reaching the insertion position or immediately before the sensor unit carrier 130 reaches the insertion position.

At one end portion of the second movement space 1312 adjacent to the handle housing 120, a sensor unit carrier ring portion 1316 to which an elastic member 150 to be described later is fixedly disposed may be provided. Meanwhile, on one surface of the sensor unit carrier body 1310 opposite the second movement space 1312, a fixing groove 1340 having a shape corresponding to the fixing protrusion portion 1137 may be formed to be recessed, and it may be preferable that the fixing groove 1340 is formed to be recessed at a position corresponding to the fixing protrusion portion 1137 when the sensor unit carrier 130 is positioned at an initial position.

### Needle carrier

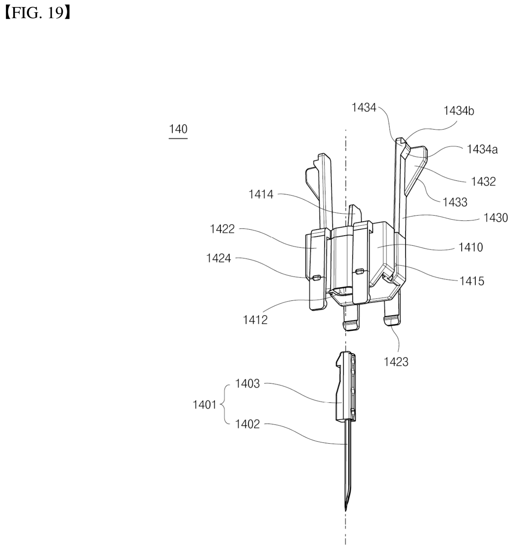

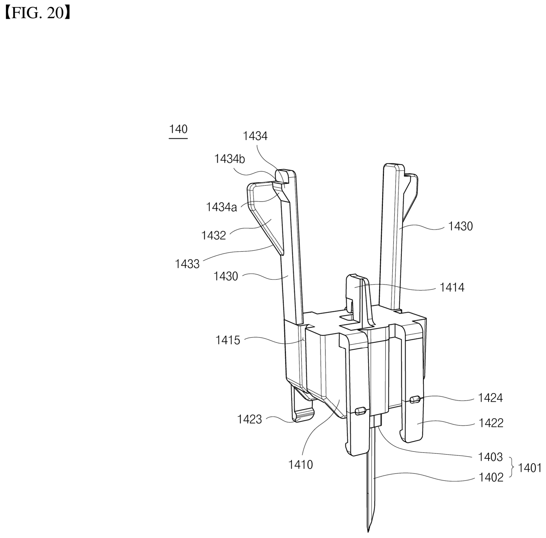

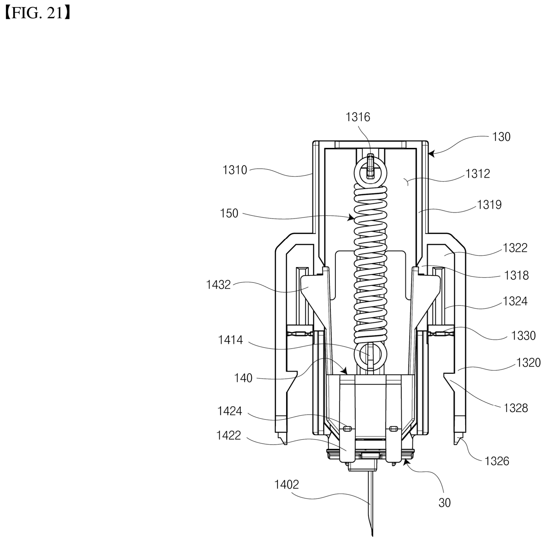

FIG. 19 is a perspective view exemplarily illustrating a coupling relationship of the needle carrier 140, FIG. 20 is a perspective view illustrating an exemplary embodiment of the needle carrier 140, and FIG. 21 is a front view exemplarily illustrating a coupling relationship between the needle carrier 140 and the sensor unit carrier 130.

The needle carrier 140 may be configured to move along the first direction together with the sensor unit carrier 130 and the sensor unit 30 by being provided with the needle 1401 for the insertion of the transcutaneous sensor member 330 into the subcutaneous tissue, and may be configured to move in a direction opposite to the first direction in the second movement space 1312 after the insertion of the transcutaneous sensor member 330 into the subcutaneous tissue to remove the needle body 1402 from the subcutaneous tissue.

The needle carrier 140 may include the needle 1401 and the needle carrier body 1410. The needle carrier body 1410 may fix the needle 1401 and be configured to move together with the needle 1401. The needle 1401 may include the needle body 1402 provided such that one end thereof is inserted into the subcutaneous tissue for the insertion of the transcutaneous sensor member 330 into the subcutaneous tissue, and a needle holder 1403 for fixing the needle body 1402. At one end of the needle carrier body 1410 facing the fourth opening 1312', a needle holder insertion groove 1412 into which the needle holder 1403 is inserted and fixed may be formed to be recessed. By the needle holder 1403 being inserted and fixed in the needle holder insertion groove 1412, the needle 1401 may be fixed to the needle carrier body 1410. When the needle carrier 140 is positioned at an initial position and an insertion position, the needle body 1402 may be discharged to the exterior through the fourth opening 1312', and when the needle carrier 140 is restored to a retracted position, the needle body 1402 may be introduced through the fourth opening 1312' and disposed in the interior of the second movement space 1312.

On one side surface of the needle carrier body 1410, the needle guide groove 1415 having a shape recessed from one side surface of the needle carrier body 1410 and extending along the first direction may be provided. A leading end portion of the above-described needle carrier guide protrusion 1313 may be disposed in the interior of the needle guide groove 1415, and by interaction between the needle carrier guide protrusion 1313 and the needle guide groove 1415, the movement direction of the needle carrier 140 in which the needle carrier 140 moves in a direction opposite to the first direction may be guided.

A needle carrier wing body 1430 may be provided in a pair in a shape extending toward a direction opposite to the first direction from both side ends of the needle carrier body 1410. The needle carrier wing body 1430 may be deformed upon application of an external force and may be provided to be restored to an original state after removal of the external force. At a leading end portion of the needle carrier wing body 1430, a needle carrier latch 1434 and a trigger 1432 may be provided. The needle carrier latch 1434 may include a constraint surface 1434b formed in a direction substantially parallel to the second direction, and an inclined surface 1434a inclinedly disposed to become closer to the needle carrier wing body 1430 toward the first direction from one end of the protruded constraint surface 1434b. The trigger 1432 may be provided to protrude outward relative to the needle carrier latch 1434. The trigger 1432 may be provided with a trigger inclined portion 1433 so as to have a shape in which a cross-section of the trigger 1432 decreases along the first direction. The trigger 1432 may preferably be disposed at the outside of the second movement space 1312.

Before operation of the applicator assembly 1 or when the needle carrier 140 moves along the first direction, since the constraint surface 1434b of the needle carrier latch 1434 maintains a state of being in contact with the detent constraint surface 1318b of the sensor unit carrier detent 1318, relative movement of the needle carrier 140 with respect to the sensor unit carrier 130 may be restricted. That is, in a state where the constraint surface 1434b of the needle carrier latch 1434 is in contact with the detent constraint surface 1318b of the sensor unit carrier detent 1318, independent movement of the needle carrier 140 is impossible, and the needle carrier 140 may move together only when the sensor unit carrier 130 moves in a state in which the needle carrier 140 is constrained to the sensor unit carrier 130. When the needle carrier 140 moves to a position adjacent to the insertion position in the insertion process of the transcutaneous sensor member 330 into the subcutaneous tissue, the trigger inclined portion 1433 of the trigger 1432 comes into contact with a leading end portion of the partition wall 1112, so that the needle carrier wing body 1430 may be deformed toward the inside of the second movement space 1312. As the needle carrier wing body 1430 is deformed toward the inside of the second movement space 1312, the contact constraint of the constraint surface 1434b of the needle carrier latch 1434 with the detent constraint surface 1318b of the sensor unit carrier detent 1318 may be released. By releasing the contact constraint of the constraint surface of the needle carrier latch 1434 from the detent constraint surface 1318b of the sensor unit carrier detent 1318, the needle carrier latch 1434 may ride over the sensor unit carrier detent 1318, and by an elastic force applied from the elastic member 150, the needle carrier 140 may move in a direction opposite to the first direction and reach a retracted position. To achieve purposes such as preventing pain due to excessive insertion of the human body B of the needle body 1402, and the like, it may be preferable that movement constraint of the sensor unit carrier 130 with respect to the needle carrier 140 be released before the sensor unit carrier 130 reaches the insertion position. In this case, even if the needle carrier 140 moves in the direction opposite to the first direction, the sensor unit carrier 130 maintains a state of pressing the sensor unit 30 toward the first direction, so that the transcutaneous sensor member 330 may be accurately inserted into an intended subcutaneous position by the inherent rigidity of the transcutaneous sensor member 330.

A plurality of grip arms 1422 may be provided in a shape extending along the first direction from other both side ends of the needle carrier body 1410. At a leading end portion of the grip arm 1422, a grip portion 1423 having a shape protruding inward may be provided, and a side end portion of the sensor unit housing 310 may be gripped by the grip portion 1423. The grip arm protrusion portion 1424 may be formed to protrude from one surface of the grip arm 1422 facing the partition wall of the column 1110, and when the needle carrier 140 is positioned at an initial position, the grip arm protrusion portion 1424 may be disposed in an interior of the grip arm guide groove 1120 before passing through the first step inclined surface 1123 or may maintain a state of being in close contact and pressed by the step portion 1121 after passing through the first step inclined surface 1123. In the insertion process of the transcutaneous sensor member 330 into the subcutaneous tissue, the grip arm protrusion portion 1424 may pass through the second step inclined surface 1124, and the close-contact pressing of the grip arm protrusion portion 1424 by the step portion 1121 may be released, and in a state where each of the grip arms 1422 is somewhat spread outward, the sensor unit 30 may be delivered to the transmission unit 40.

At one end of the needle carrier body 1410 facing the sensor unit carrier ring portion 1316, a needle carrier ring portion 1414 may be provided, and the other end portion of the elastic member 150, of which one end is connected to the sensor unit carrier ring portion 1316, may be connected to the needle carrier ring portion 1414. The elastic member 150 may be applied without limitation as long as it is a means capable of providing a driving force to move the needle carrier 140 in a direction opposite to the first direction, but a tension spring may be preferably applied.

### Cap

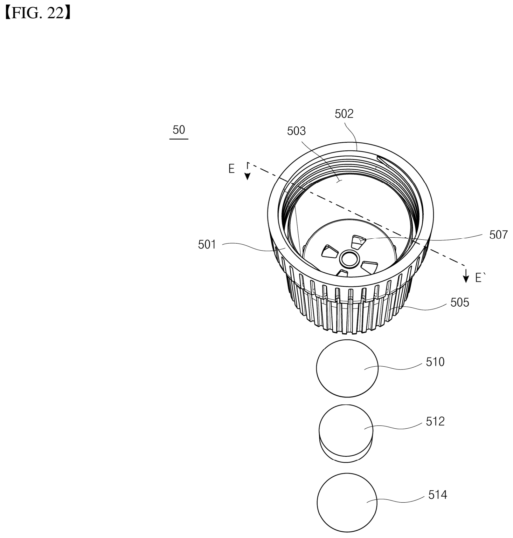

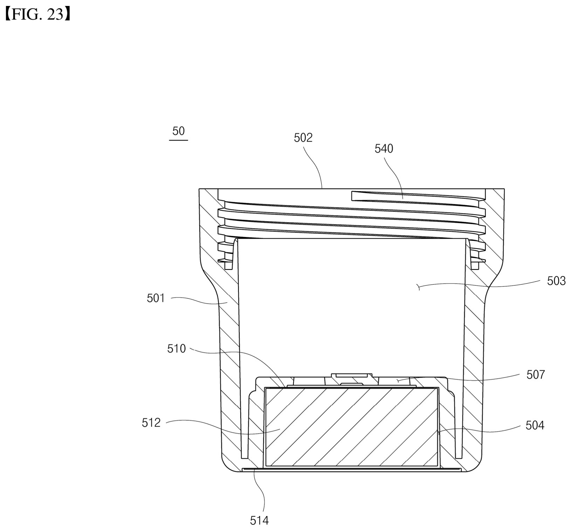

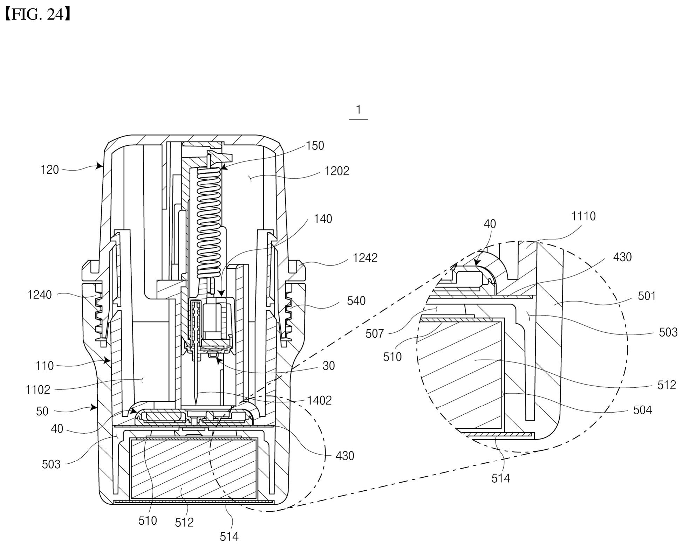

FIG. 22 is an exploded perspective view illustrating an exemplary embodiment of the cap 50, FIG. 23 is a cross-sectional view of the cap 50 cut along the E-E' direction in FIG. 22. FIGS. 24 to 26 are partially enlarged cross-sectional views illustrating an exemplary embodiment of the applicator assembly 1 to which the cap 50 is applied.

The cap 50 may be fixedly disposed so as to be releasable at one end of the applicator 10. Since the applicator assembly 1 includes the cap 50, it may effectively prevent unintended arbitrary firing of the applicator 10 or introduction of external contaminants or moisture into the interior of the applicator assembly 1, which may occur in manufacturing, distribution, storage, and use processes of the applicator assembly 1.

The cap 50 may include a cap housing 501 forming an external shape of the cap 50. In an interior of the cap housing 501, an accommodation space 503 communicating with the exterior through a fifth opening 502 formed at one end of the cap housing 501 may be formed. On an inner surface at one end of the cap housing 501 where the fifth opening 502 is formed, the screw threaded portion 540 having a shape corresponding to the screw threaded portion 1240 formed on the handle housing 120 may be provided. As the screw threaded portion 1240 formed on the handle housing 120 is screw-coupled to the screw threaded portion 540 formed on the cap housing 501, the cap 50 may be separably screw-coupled to one end of the handle housing 120. On an outer surface of the cap housing 501, a plurality of grip grooves 505 may be formed to be recessed in order to facilitate the work by a worker or user or the convenience of use.

Meanwhile, although a cup-shaped cap housing 501 is illustrated in the drawings, the shape of the cap housing 501 of the present invention is not necessarily limited thereto, and the shape of the cap housing 501 may be variously modified and applied as long as it can protect and dehumidify the interior of the applicator 10. The cap housing 501 may be means for protecting and dehumidifying the interior of the applicator 10. However, since one end of the cap housing 501 in which the screw threaded portion 540 is formed is intended to be screw-coupled to the screw threaded portion 1240 formed on the handle housing 120, it may be preferable that the end portion of the cap housing 501 in which the screw threaded portion 540 is formed be provided in a cylindrical shape corresponding to the end portion of the handle housing 120.

At the other closed end portion of the cap housing 501 facing the fifth opening 502, an accommodation groove 504 having a shape recessed from the outside toward the accommodation space 503 may be provided. At the other end portion of the cap housing 501 forming the accommodation groove 504, a plurality of ventilation holes 507 communicating the accommodation groove 504 with the accommodation space 503 may be formed to penetrate. In the accommodation groove 504, a first sealing member 510, a dehumidifier 512, and a second sealing member 514 may be sequentially disposed from one side of the ventilation holes 507 toward the outside. The first sealing member 510 may preferably be manufactured using a material that allows air to pass through but does not allow moisture to pass through, and as a non-limiting example, Tyvek material of DuPont may be used. By means of the first sealing member 510, introduction of moisture into the accommodation space 503 may be effectively prevented. The dehumidifier 512 may preferably be manufactured using a material having a dehumidifying function applied to an electronic device or a medical device. By means of the dehumidifier 512, moisture introduced into the accommodation space 503 may be removed. The second sealing member 514 may be disposed so as to seal the accommodation groove 504 from the exterior, and may be manufactured by applying a material that prevents the passage of moisture and external contamination sources. As a non-limiting example, the second sealing member 514 may be manufactured using an aluminum packaging sheet. Since the first sealing member 510, the dehumidifier 512, and the second sealing member 514 are sequentially disposed in the accommodation groove 504 communicating with the accommodation space 503 through the ventilation holes 507, not only can moisture introduced into the interior of the accommodation space 503 be effectively removed, but also introduction of contaminants or moisture from the exterior into the interior of the accommodation space 503 can be effectively prevented.

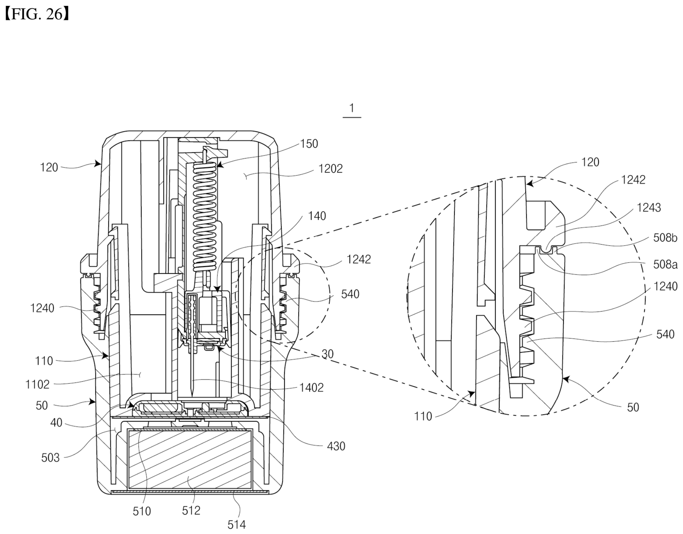

As illustrated in FIG. 26, sealing members 508a and 508b, which are disposed upright in a ring shape, may be provided in a pair at a leading end portion of the cap housing 501, and a calking member 1243 may be protrudingly formed on one surface of the locking protrusion portion 1242 corresponding to the leading end portion of the cap housing 501. When the screw threaded portion 540 formed in the cap housing 501 and the screw threaded portion 1240 formed in the handle housing 120 are screw-coupled to each other such that the cap 50 is coupled to one end of the handle housing 120, the sealing members 508a and 508b may closely contact and press the calking member 1243 to effectively prevent introduction of external contaminants or moisture into the space between the handle housing 120 and the cap 50. The calking member 1243 and the sealing members 508a and 508b may include a case where they are integrally injection-molded together with the handle housing 120 and the cap housing 501, or a case where they are manufactured using a material having inherent elasticity such as rubber or silicone.

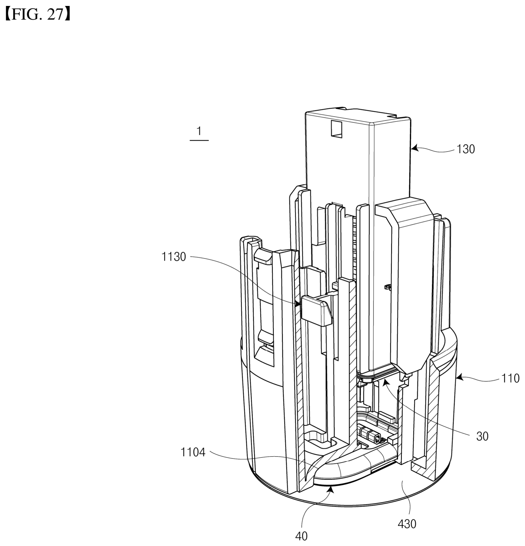

Hereinafter, with reference to FIGS. 27 to 46, the assembly and operation processes of the applicator 10 and the applicator assembly 1 will be described in more detail.

### Exemplary embodiment of action of fixing portion

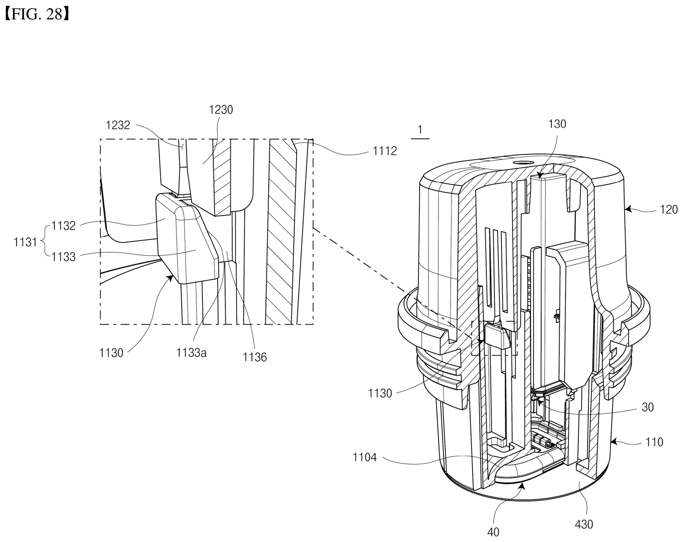

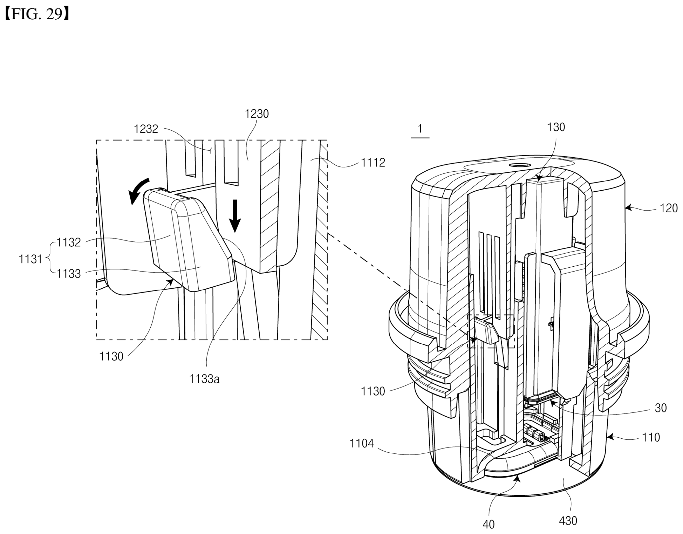

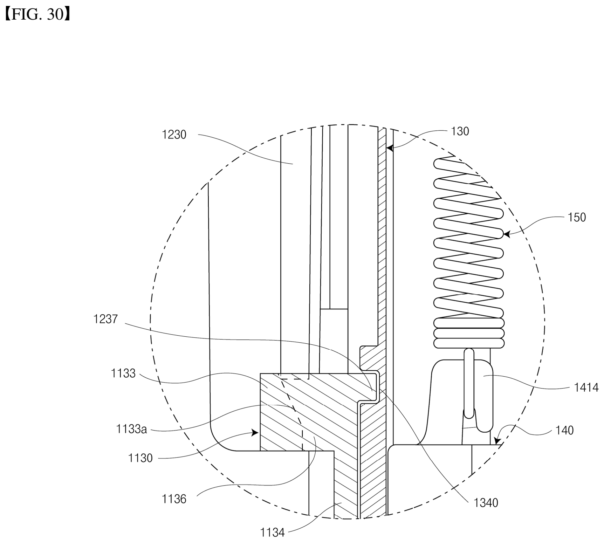

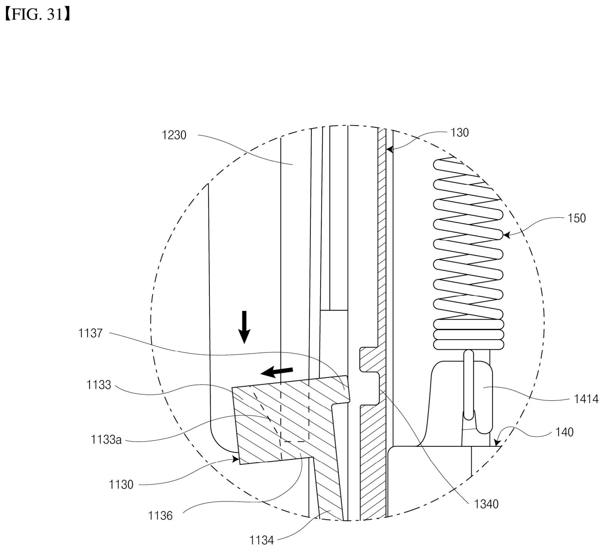

FIG. 27 is a partial cross-sectional view illustrating an exemplary embodiment in a state where the sensor unit carrier 130 is coupled to the body housing 110 during an assembly process of the applicator assembly 1, and FIGS. 28 and 29 are partial cross-sectional views illustrating an exemplary embodiment of a process of coupling the handle housing 120 to the body housing 110 to which the sensor unit carrier 130 is coupled during the assembly process of the applicator assembly 1. FIG. 30 is an enlarged cross-sectional view illustrating an exemplary embodiment in a state where a leading end portion of the fixing protrusion portion 1137 is inserted into the fixing groove 1340 during the assembly process of the applicator assembly 1, and FIG. 31 is an enlarged cross-sectional view illustrating an exemplary embodiment in a state where the leading end portion of the fixing protrusion portion 1137 is discharged from the fixing groove 1340 during the assembly process of the applicator assembly 1.

As illustrated in FIGS. 27 and 30, in a process of assembling the body housing 110 and the sensor unit carrier 130, the sensor unit carrier body 1310 may be fitted into the column 1110 forming the first movement space 1111. In the process of fitting the sensor unit carrier body 1310 into the column 1110, the fixing protrusion portion 1137 may come into contact with one surface of the sensor unit carrier body 1310, and the support 1134 may maintain a state deformed outward. As the sensor unit carrier body 1310 moves along the first direction, the support 1134 may maintain the state deformed outward, and as the fixing groove 1340 formed in the sensor unit carrier body 1310 reaches a position corresponding to the fixing protrusion portion 1137, the support 1134 may be restored to an original state from the deformed state. That is, as the leading end portion of the fixing protrusion portion 1137 is introduced into the fixing groove 1340, the pressing movement portion 1131 may move toward the sensor unit carrier body 1310, and the support 1134 may also be restored from deformation.

As the leading end portion of the fixing protrusion portion 1137 is introduced into the fixing groove 1340, movement of the sensor unit carrier 130 in the first direction may be restricted. In the assembly process of the applicator assembly 1, a worker may recognize, through a sound generated by parts hitting each other or a sensation transmitted to fingertips, that the leading end portion of the fixing protrusion portion 1137 has been normally introduced into the fixing groove 1340.

Subsequently, the worker may perform a work of coupling the handle housing 120 to the body housing 110 to which the sensor unit carrier 130 is coupled. When the handle housing 120 is coupled to the body housing 110 to which the sensor unit carrier 130 is coupled, the sensor unit carrier 130 may be pressed by the handle housing 120 entering for assembly, thereby causing an arbitrary firing or causing the sensor unit carrier 130 to deviate from the initial position. However, when the assembly of the handle housing 120 is performed in a state in which the leading end portion of the fixing protrusion portion 1137 is introduced into the fixing groove 1340, an arbitrary firing of the applicator 10 or a positional deviation of the sensor unit carrier 130 may be effectively prevented.

As illustrated in FIGS. 28, 29, and 31, in the process of coupling the handle housing 120 to the body housing 110 to which the sensor unit carrier 130 is coupled, the leading end portion of the push arm 1230 provided in the handle housing 120 moves to a position in contact with the pressing movement inclined surface 1133a of the pressing movement wing portion 1133. As the assembly of the handle housing 120 proceeds, the leading end portion of the push arm 1230, in a state of being in contact with the pressing movement inclined surface 1133a, move in the first direction to press the pressing movement wing portion 1133, and the support 1134 is deformed outward, whereby the pressing movement portion 1131 is pushed in a direction away from the sensor unit carrier body 1310. As the pressing movement portion 1131 is pushed in the direction away from the sensor unit carrier body 1310, the leading end portion of the fixing protrusion portion 1137 may be discharged from the fixing groove 1340. As the leading end portion of the fixing protrusion portion 1137 is discharged from the fixing groove 1340, arbitrary movement restriction of the sensor unit carrier 130 by the fixing portion 1130 is released, and the sensor unit carrier 130 may maintain a state of being able to move irrespective of the fixing portion 1130.

### Exemplary embodiment of operation of needle carrier

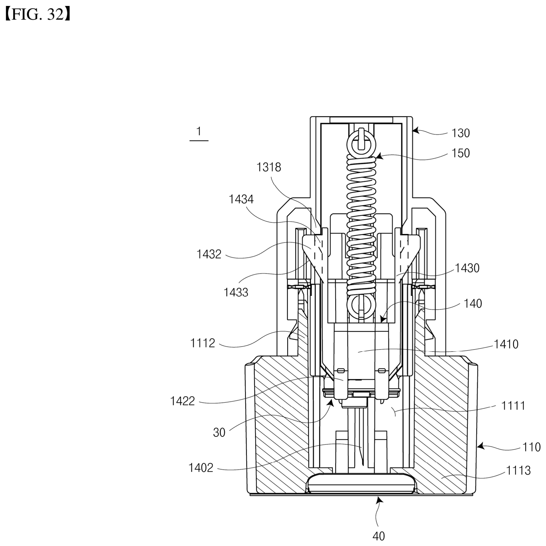

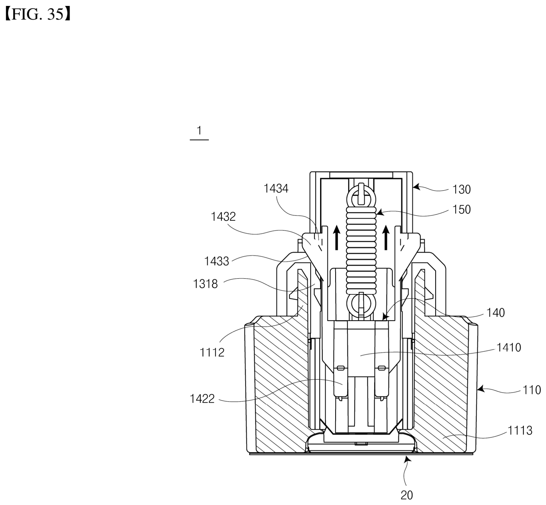

FIGS. 32 to 34 are partial cross-sectional views sequentially illustrating an exemplary embodiment of a state in which the sensor unit carrier 130 moves from an initial position to an insertion position during an operation process of the applicator assembly 1, and FIG. 35 is a partial cross-sectional view illustrating an exemplary embodiment of a state in which the needle carrier 140 moves to a retracted position during the operation process of the applicator assembly 1.

As illustrated in FIG. 32, in a process in which the sensor unit carrier 130 moves from the initial position toward the insertion position, since the constraint surface 1434b of the needle carrier latch 1434 maintains a state of being in contact with the detent constraint surface 1318b of the sensor unit carrier detent 1318, the needle carrier 140 may maintain a state constrained to the sensor unit carrier 130 and may move along the first direction together with the sensor unit carrier 130.

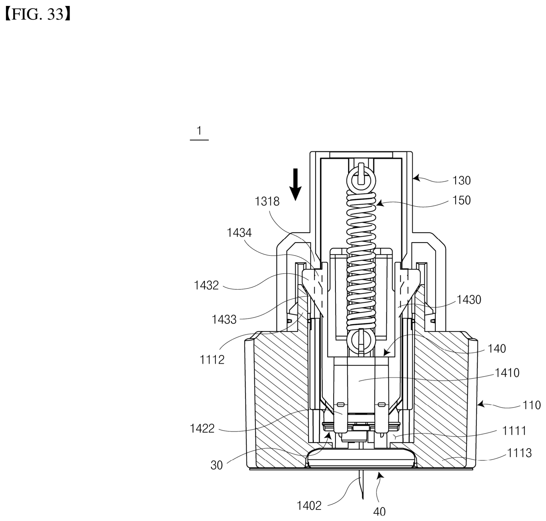

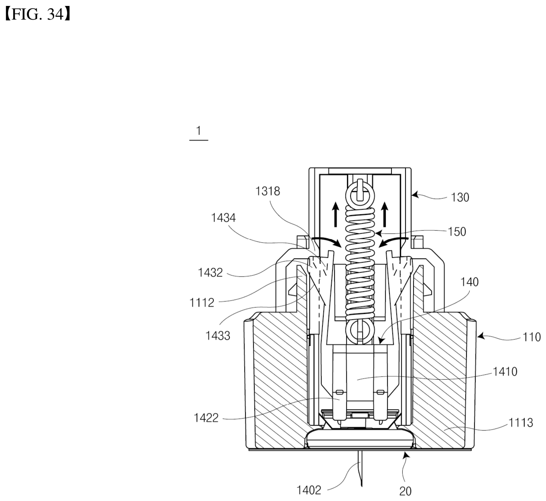

As illustrated in FIG. 33, when the sensor unit carrier 130 reaches a position adjacent to the insertion position, the trigger inclined portion 1433 of the trigger 1432 comes into contact with a leading end portion of the partition wall 1112. Meanwhile, as illustrated in FIG. 34, as the sensor unit carrier 130 continuously moves in the first direction, the needle carrier wing body 1430 is deformed toward the inside of the second movement space 1312, and accordingly, the constraint surface 1434b of the needle carrier latch 1434 and the detent constraint surface 1318b of the sensor unit carrier detent 1318 may deviate from a position of being in mutual contact. Therefore, the needle carrier latch 1434 becomes released from the constraint of the sensor unit carrier detent 1318, and the needle carrier 140 may relatively move with respect to the sensor unit carrier 130. Meanwhile, as illustrated in FIGS. 33 and 34, the release of the movement constraint of the needle carrier 140 by the sensor unit carrier 130 may be performed before the sensor unit carrier 130 reaches the insertion position, and accordingly, side effects due to excessive human body B insertion of the needle body 1402 can be effectively prevented.

As illustrated in FIG. 35, after the constraint of the needle carrier latch 1434 by the sensor unit carrier detent 1318 is released, the needle carrier 140 may relatively move with respect to the sensor unit carrier 130, and by a driving force applied from the elastic member 150, the needle carrier 140 may move in a direction opposite to the first direction to reach the retracted position. When the needle carrier 140 reaches the retracted position, since the leading end portion of the needle body 1402 is disposed so as to be entirely accommodated in the first movement space 1111, a state in which the needle body 1402 is no longer exposed or protruded to the exterior of the applicator 10 may be maintained.

### Exemplary embodiment of action of bridge

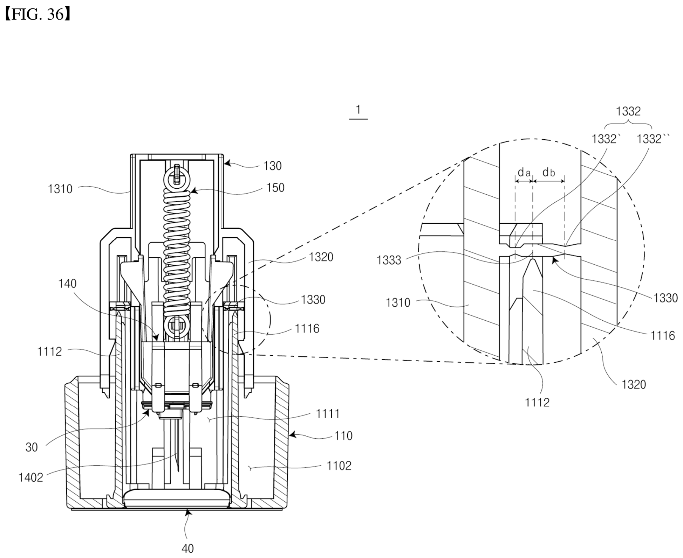

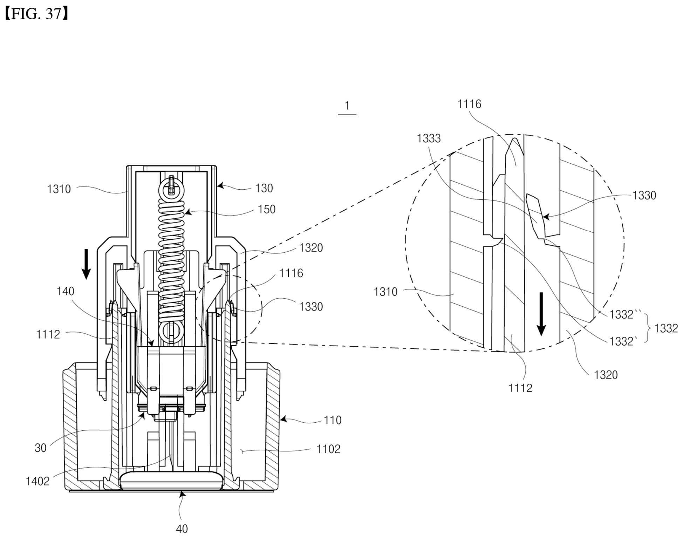

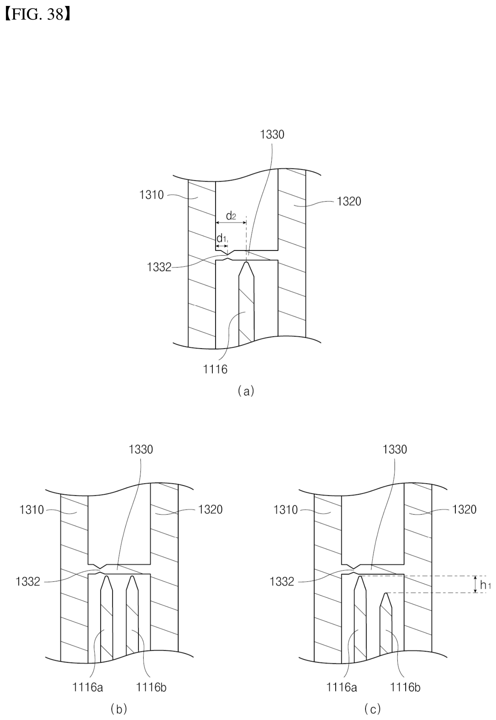

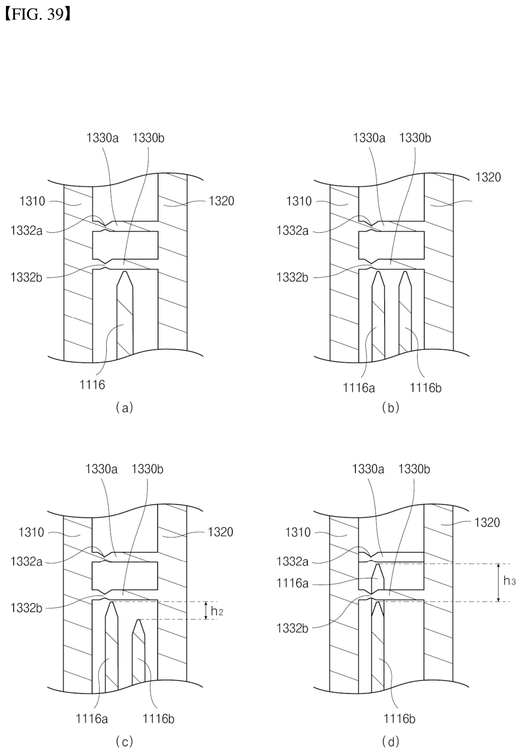

FIG. 36 is a partial cross-sectional view illustrating an exemplary embodiment of a state before the bridge 1330 is cut during an operation process of the applicator assembly 1, and FIG. 37 is a partial cross-sectional view illustrating an exemplary embodiment of a state in which the bridge 1330 is cut during the operation process of the applicator assembly 1. FIGS. 38 and 39 are partial cross-sectional views illustrating an exemplary embodiment of a deformable bridge and a bridge pressing portion.

Hereinafter, with reference to FIG. 12, FIGS. 15 to 18, and FIGS. 36 to 39, the specific configuration of the bridge 1330 and the bridge pressing portion 1116, and the mutual interaction between the bridge 1330 and the bridge pressing portion 1116 will be described in more detail.

As illustrated in FIGS. 17 and 18, the bridge 1330 may be disposed to connect the extension arm 1320 and the sensor unit carrier body 1310 in a space between the extension arm 1320 and the sensor unit carrier body 1310. That is, one end and the other end of the bridge 1330 may be respectively connected to one side surface of the sensor unit carrier body 1310 and one side surface of the extension arm 1320 facing the sensor unit carrier body 1310. The bridge 1330 may be disposed between the extension arm connection portion 1322 and the second acceleration latch 1328. During assembly of the applicator assembly 1, one end of the sensor unit carrier body 1310 may be introduced into the first movement space 1111 formed in the interior of the column 1110, and as the sensor unit carrier body 1310 is continuously introduced into the first movement space 1111, the bridge 1330 may be disposed at a position facing the bridge pressing portion 1116 provided in the column 1110. Hereinafter, a case in which the bridge 1330 is disposed to be spaced apart at a predetermined interval from the bridge pressing portion 1116 before firing of the applicator assembly 1 will be described as one example, but a state in which the bridge 1330 is in contact with the bridge pressing portion 1116 before firing of the applicator assembly 1 may be included.

The bridge 1330 may have one or more vulnerable portions 1332 formed therein to have a relatively small thickness. The vulnerable portion 1332 is a region where cutting by the bridge pressing portion 1116 is intended, and is preferably designed so that the vulnerable portion 1332 is cut only when a force by which a user presses the handle housing 120 is delivered with the purpose of inserting the transcutaneous sensor member 330 into subcutaneous tissue. Here, the force by which a user presses the handle housing 120 with the purpose of inserting the transcutaneous sensor member 330 into subcutaneous tissue does not mean a level of force that is normally applied to the handle housing 120 in the assembly or transportation process of the applicator assembly 1, but is preferably interpreted as a level of force for pressing the handle housing 120 by the user of the applicator assembly 1 in a state of intending firing of the transcutaneous sensor member 330. Meanwhile, in the above, a region having a smaller thickness compared to the average thickness of the bridge 1330 has been described as one example of the vulnerable portion 1332, but the vulnerable portion 1332 is not necessarily limited to a structure having a relatively small thickness, and may be interpreted as meaning a region having a lower breaking strength compared to the entire bridge 1330 by applying heterogeneous materials or the like or applying a vulnerable structure.

As illustrated in FIG. 12, FIG. 15, and FIG. 16, at a leading end portion of the partition wall 1112, the bridge pressing portion 1116 having one or more inclined surfaces and having a shape in which a cross-section decreases toward the leading end may be disposed. During assembly of the applicator assembly 1, as the sensor unit carrier body 1310 is introduced into the first movement space 1111, at least one end portion of the carrier guide protrusion 1310a may be disposed in the interior of the carrier guide groove 1114, and accordingly, the bridge 1330 may be disposed at a position facing the bridge pressing portion 1116. Meanwhile, in the partition wall 1112 where the bridge pressing portion 1116 is disposed, a carrier slit 1117 formed by incision along the first direction is provided at a position adjacent to the bridge pressing portion 1116, and in the extension arm connection portion 1322 disposed between the carrier body 1310 and the extension arm 1320, an extension arm guide groove 1324 recessed in a shape corresponding to the carrier slit 1117 is formed to extend along the first direction, so that in the pressing process of the bridge 1330 by the bridge pressing portion 1116, deviation of the bridge 1330 from a position corresponding to the bridge pressing portion 1116 can be effectively prevented. That is, in a process in which the sensor unit carrier 130 moves along the first direction, at least one end of the extension arm guide groove 1324 is introduced into an interior of the carrier slit 1117 to guide the movement of the sensor unit carrier body 1310 in the first direction, thereby effectively preventing the bridge 1330 from being deviated from the leading end portion of the bridge pressing portion 1116 in the insertion process of the transcutaneous sensor member 330 into the subcutaneous tissue.

In FIG. 12 and FIGS. 15 to 18, a case is exemplarily illustrated in which, after pressing of the bridge 1330 by the bridge pressing portion 1116 is initiated in a process in which the sensor unit carrier 130 moves along the first direction, the bridge 1330 is disposed at a position in which at least one end of the extension arm guide groove 1324 is introduced into the carrier slit 1117. However, it may include a case in which the bridge 1330 is disposed at a position where at least one end of the extension arm guide groove 1324 is introduced into the carrier slit 1117 immediately before pressing of the bridge 1330 by the bridge pressing portion 1116 or simultaneously with pressing of the bridge 1330 by the bridge pressing portion 1116.

As illustrated in FIG. 36, when the sensor unit carrier 130 is positioned at an initial position, the bridge 1330 may be disposed in contact with the bridge pressing portion 1116 or at a position spaced apart at a predetermined interval from the bridge pressing portion 1116.

As illustrated in FIG. 37, when the sensor unit carrier 130 moves in the first direction for the insertion of the transcutaneous sensor member 330 into the subcutaneous tissue, the bridge 1330 moves to a position in contact with and pressed by the bridge pressing portion 1116, and the bridge 1330 may be broken only when a force greater than the designed breaking strength is applied to the bridge 1330. That is, firing of the transcutaneous sensor member 330 is performed only when a force greater than the designed breaking strength of the bridge 1330 is applied to the handle housing 120, so that the needle body 1402 may be inserted into the subcutaneous tissue together with the transcutaneous sensor member 330 in a state where sufficient acceleration conditions are granted.

Meanwhile, when the bridge 1330 is not broken despite the movement of the sensor unit carrier 130 in the first direction, a state in which the bridge 1330 is caught by the bridge pressing portion 1116 is maintained, and the sensor unit carrier 130 is placed in a situation in which further movement in the first direction is impossible. That is, the bridge 1330 and the bridge pressing portion 1116 permit the insertion of the transcutaneous sensor member 330 into the subcutaneous tissue only when a force of a predetermined level or more is applied to the handle housing 120, thereby effectively preventing a user's unintended arbitrary firing of the transcutaneous sensor member 330 by the mutual interaction between the bridge 1330 and the bridge pressing portion 1116.

As illustrated in FIGS. 36 and 37, the bridge 1330 may be provided with a first vulnerable portion 1332' and a second vulnerable portion 1332" disposed spaced apart from each other. Between the first vulnerable portion 1332' and the second vulnerable portion 1332", a contact portion 1333, in which contact with a leading end portion of the bridge pressing portion 1116 is intended as the sensor unit carrier 130 moves in the first direction, may be provided. When the sensor unit carrier 130 moves in the first direction, after the leading end portion of the bridge pressing portion 1116 comes into contact with a specific region of the bridge 1330, the bridge 1330 may be deformed so that the leading end portion of the bridge pressing portion 1116 comes into contact with various regions of the bridge 1330. However, the contact portion 1333 may mean a region of the bridge 1330 with which the leading end portion of the bridge pressing portion 1116 first comes into contact as the sensor unit carrier 130 moves in the first direction.

The first vulnerable portion 1332' and the second vulnerable portion 1332" may have a relatively small average thickness compared to the average thickness of the bridge 1330. The first vulnerable portion 1332' may have a smaller thickness compared to the second vulnerable portion 1332". A first direction thickness of the first vulnerable portion 1332' may be smaller compared to a first direction thickness of the second vulnerable portion 1332". A second direction thickness of the first vulnerable portion 1332' may be smaller compared to a second direction thickness of the second vulnerable portion 1332". Since the first vulnerable portion 1332' is provided to have a smaller thickness compared to the second vulnerable portion 1332", when the leading end portion of the bridge pressing portion 1116 presses the contact portion 1333, breakage may occur in the first vulnerable portion 1332' and bending may occur in the second vulnerable portion 1332". Depending on the difference in thickness between the first vulnerable portion 1332' and the second vulnerable portion 1332", and on formation positions of the first vulnerable portion 1332' and the second vulnerable portion 1332", a threshold value of force to be applied to the applicator assembly 1 for the sensor unit carrier 130 to move from an initial position to an insertion position may be variably adjusted.

The first vulnerable portion 1332' may be disposed relatively adjacent to the sensor unit carrier body 1310 compared to the second vulnerable portion 1332", and the contact portion 1333 may be disposed more adjacent to the first vulnerable portion 1332' compared to the second vulnerable portion 1332" (da < db). When the leading end portion of the bridge pressing portion 1116 presses the contact portion 1333 and breakage occurs in the first vulnerable portion 1332', the contact portion 1333 may be pushed toward a direction opposite to the first direction compared to the first vulnerable portion 1332' and the second vulnerable portion 1332", and accordingly, the bridge pressing portion 1116 and the second vulnerable portion 1332" may be disposed at the outside of the partition wall 1112. When the first vulnerable portion 1332' is broken, the contact portion 1333 is disposed in an outside region of the partition wall 1112, thereby effectively preventing fragments formed in a cutting process of the bridge 1330 from falling toward the sensor unit 30 or the transmission unit 40. That is, the occurrence of poor coupling or malfunction of the wearable unit 20, which may be caused by the introduction of the fragments of the bridge 1330, which are formed in the insertion process of the transcutaneous sensor member 330 into the subcutaneous tissue, into the coupling portion of the sensor unit 30 and the transmission unit 40, may be more effectively prevented.

Hereinafter, with reference to FIGS. 38 and 39, the exemplary embodiment and modified embodiment of the bridge 1330 and the bridge pressing portion 1116 will be described in more detail.

FIG. 38(a) is an enlarged partial cross-sectional view illustrating the bridge 1330 and the bridge pressing portion 1116 in a region indicated by a circular dotted line in FIG. 36. As illustrated in FIG. 38(a), the vulnerable portion 1332 may preferably be disposed closer to the sensor unit carrier body 1310 compared to the leading end portion of the bridge pressing portion 1116. That is, a distance (d1) from one surface of the sensor unit carrier body 1310 to the vulnerable portion 1332 to which the bridge 1330 is connected may be shorter compared to a distance (d2) from one surface of the sensor unit carrier body 1310 to the leading end portion of the bridge pressing portion 1116 to which the bridge 1330 is connected. As the sensor unit carrier 130 moves in the first direction, the bridge 1330 is cut, and a piece of the cut bridge 1330 may be introduced into the first movement space 1111, thereby interfering with the normal insertion of the transcutaneous sensor member 330 into the subcutaneous tissue or human body discharge of the needle body 1402 and potentially causing a serious safety problem. When the vulnerable portion 1332 is formed at a position closer to one surface of the sensor unit carrier body 1310 compared to the leading end portion of the bridge pressing portion 1116 (d1 < d2), even if breakage occurs at the vulnerable portion 1332, the bridge 1330 connected to the extension arm 1320 maintains in a state disposed at the outside of the column 1110, thereby preventing in advance a safety accident that may occur due to a piece of the cut bridge 1330 being introduced into the first movement space 1111. Meanwhile, in a process in which the sensor unit carrier 130 moves in the first direction, at least one end of the extension arm guide groove 1324 is introduced into the carrier slit 1117 formed at one side of the bridge pressing portion 1116 and partially closes the carrier slit 1117, thereby more effectively preventing a piece of the cut bridge 1330 from being introduced into the first movement space 1111 through the carrier slit 1117.

As illustrated in FIG. 38(b), one or more bridge pressing portions 1116a and 1116b may be disposed at a position facing the bridge 1330. By providing one or more bridge pressing portions 1116a and 1116b, not only may a pressing force applied to the bridge 1330 be increased, but also, even when one of the bridge pressing portions 1116a or 1116b fails to function properly due to negligence in the process of distribution or use of the applicator assembly 1, normal pressing of the bridge 1330 may be achieved by the other bridge pressing portion 1116a or 1116b.

As illustrated in FIG. 38(c), one or more bridge pressing portions 1116a and 1116b may be disposed at a position facing the bridge 1330, and these one or more bridge pressing portions 1116a and 1116b may be provided to have different leading end portion heights (Δh1). It may be more preferable that the bridge pressing portion 1116a adjacent to the sensor unit carrier body 1310 be disposed so that its leading end portion is positioned closer to the bridge 1330 compared to the bridge pressing portion 1116b adjacent to the extension arm 1320. As one or more bridge pressing portions 1116a and 1116b are disposed at a position facing the bridge 1330, not only may the pressing force applied to the bridge 1330 be effectively increased and assisted, but also the bridge pressing portion 1116a adjacent to the sensor unit carrier body 1310 may be granted with a height difference (Δh1) so that its leading end portion is disposed closer to the bridge 1330 compared to the bridge pressing portion 1116b adjacent to the extension arm 1320, thereby more effectively preventing a piece of the cut bridge 1330 from being introduced into the first movement space 1111.

As illustrated in FIG. 39(a), for granting sufficient acceleration conditions and preventing arbitrary firing, one or more bridges 1330a and 1330b may be disposed at a position facing the bridge pressing portion 1116. As illustrated in FIGS. 39(b) and 39(c), from the perspective of increasing and assisting pressing force and from the perspective of preventing introduction of a piece of the cut bridge 1330 into the first movement space 1111, one or more bridge pressing portions 1116a and 1116b may be disposed at a position facing the bridges 1330a and 1330b, or these one or more bridge pressing portions 1116a and 1116b may be provided to have different leading end portion heights (Δh2).

As illustrated in FIG. 39(d), one or more bridges 1330a and 1330b and one or more bridge pressing portions 1116a and 1116b disposed at respective positions corresponding to each of the bridges 1330a and 1330b may be included, and this may include a case where the bridges 1330a and 1330b and the bridge pressing portions 1116a and 1116b are disposed in different regions from each other. That is, it may include a case in which the first bridge 1330a may be disposed at a position facing the first bridge pressing portion 1116a, and the second bridge 1330b may be disposed at a position facing the second bridge pressing portion 1116b, but the first bridge 1330a and the second bridge 1330b are disposed in different regions from each other so that, in a process in which the sensor unit carrier 130 moves in the first direction, the first bridge pressing portion 1116a presses only the first bridge 1330a regardless of the second bridge 1330b, and the second bridge pressing portion 1116b presses only the second bridge 1330b regardless of the first bridge 1330a. In this case, a plurality of bridges 1330a and 1330b and a plurality of bridge pressing portions 1116a and 1116b are disposed in the interior of the applicator assembly 1, and since the respective bridges 1330a and 1330b and bridge pressing portions 1116a and 1116b forming pairs are disposed in different regions, not only securing spatial availability may be achieved, but also stepwise acceleration conditions may be granted through interval adjustment between the respective corresponding bridges 1330a and 1330b and bridge pressing portions 1116a and 1116b, or a stricter environment for suppressing arbitrary firing may be created.

Meanwhile, in the above, a case has been described as an example in which the bridge 1330 is disposed on the sensor unit carrier 130 and the bridge pressing portion 1116 is disposed on the body housing 110, but a case may be included in which the bridge pressing portion is disposed on the sensor unit carrier 130 and the corresponding bridge is provided on the body housing 110.

### Exemplary embodiment of interaction between first movement restriction portion and second movement restriction portion

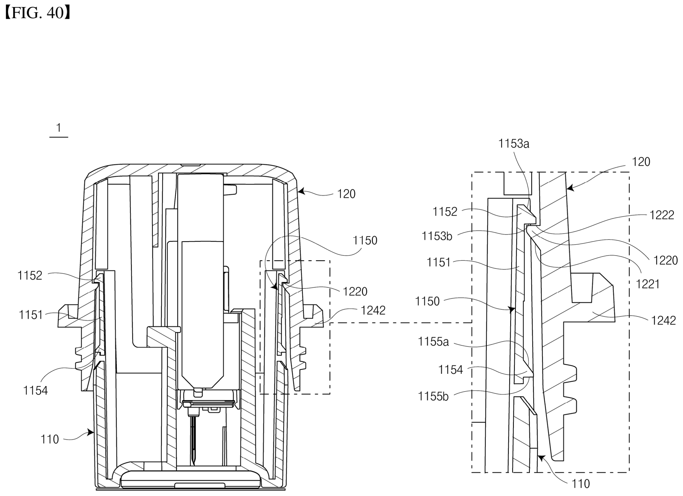

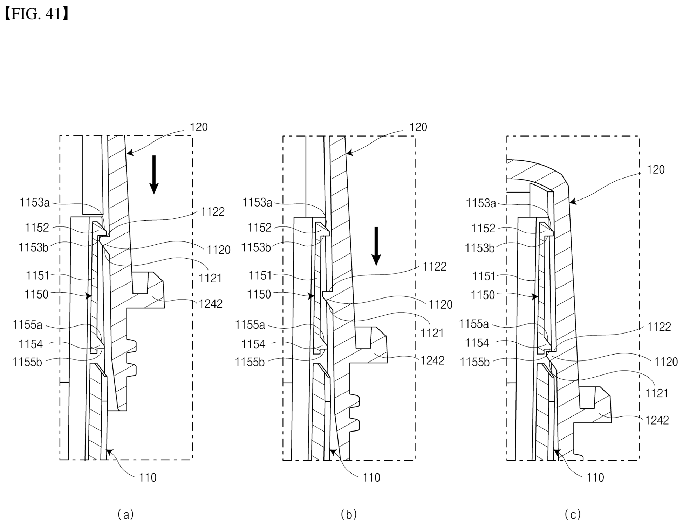

FIG. 40 is a cross-sectional view and a partial enlarged cross-sectional view for explaining an exemplary embodiment regarding a positional relationship between the first movement restriction portion 1220 and the second movement restriction portion 1150 in a state before operation of the applicator assembly 1, and FIGS. 41(a) to 41(c) are sequentially illustrated partial enlarged cross-sectional views for explaining an exemplary embodiment regarding a positional relationship between the first movement restriction portion 1220 and the second movement restriction portion 1150 during an operation process of the applicator assembly 1.

As illustrated in FIG. 40 and FIG. 41(a), in a state before operation of the applicator assembly 1, the support surface 1222 of the first movement restriction portion 1220 and the first support surface 1153b of the first movement restriction latch 1152 may be disposed to face each other. Accordingly, even when a force in a direction opposite to the first direction is applied to the handle housing 120, since the support surface 1222 of the first movement restriction portion 1220 and the first support surface 1153b of the first movement restriction latch 1152 are in contact with each other and support each other, the handle housing 120 may not further move in the direction opposite to the first direction, and thus the handle housing 120 can be effectively prevented from being arbitrarily deviated from the body housing 110.

Meanwhile, as illustrated in FIGS. 41(b) and 41(c), in a firing process of the applicator assembly 1, the inclined surface 1221 of the first movement restriction portion 1220 moves to a position adjacent to the second inclined surface 1155a of the second movement restriction latch 1154. Thereafter, in a state in which the inclined surface 1221 of the first movement restriction portion 1220 is contact with the second inclined surface 1155a of the second movement restriction latch 1154, the handle housing 120 moves in the first direction, and the other end of the movement restriction body 1151 is deformed toward the second interior space 1102, so that the first movement restriction portion 1220 passes through the second movement restriction latch 1154. After the first movement restriction portion 1220 passes through the second movement restriction latch 1154, the movement restriction body 1151 is restored to a state before deformation, and the support surface 1222 of the first movement restriction portion 1220 and the second support surface 1155b of the second movement restriction latch 1154 are placed in a state facing each other. Since the movement of the handle housing 120 in a direction opposite to the first direction is restricted by the interaction between the support surface 1222 of the first movement restriction portion 1220 and the second support surface 1155b of the second movement restriction latch 1154, reuse of the applicator 10 after firing of the transcutaneous sensor member 330 can be strictly restricted.

### Exemplary embodiment of action of grip arm protrusion portion

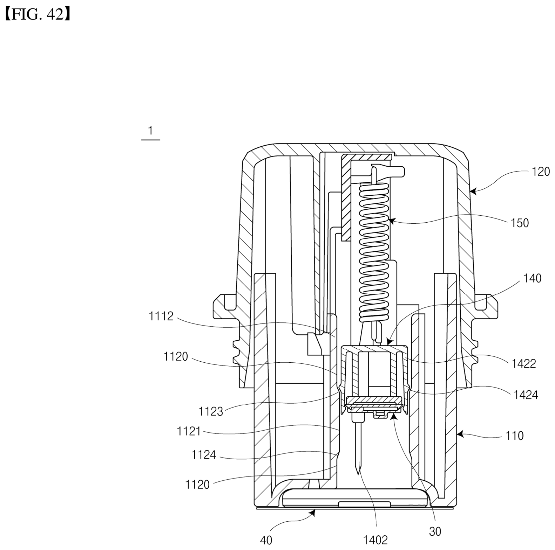

FIGS. 42 to 44 are sequentially illustrated cross-sectional views for explaining an exemplary embodiment of pressing and pressing release of the grip arm protrusion portion 1424 during an operation process of the applicator assembly 1.

FIG. 42 is a view illustrating a state before operation of the applicator assembly 1, and the grip arm protrusion portion 1424 may be disposed in a state of not having passed through the first step inclined surface 1123. That is, since the grip arm protrusion portion 1424 maintains a state of not being pressed by the step portion 1121, the grip arm 1422 may grip the sensor unit 30 in a somewhat loose state. Meanwhile, unlike FIG. 42, the grip arm protrusion portion 1424 may be disposed in a state of having passed through the first step inclined surface 1123 in the state before operation of the applicator assembly 1, and in this case, the grip arm protrusion portion 1424 is pressed by the step portion 1121 so that the grip arm 1422 may maintain a state of gripping the sensor unit 30 firmly.

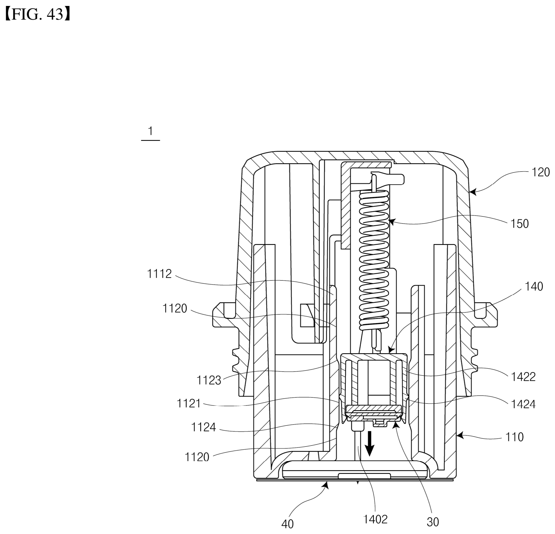

When the handle housing 120 is pressed in the first direction by a user, as illustrated in FIG. 43, the grip arm protrusion portion 1424 maintains a state of being pressed by the step portion 1121 and moves in the first direction, and in this process, the sensor unit 30 maintains a state of being firmly gripped by the grip arm 1422 and may move toward the transmission unit 40.

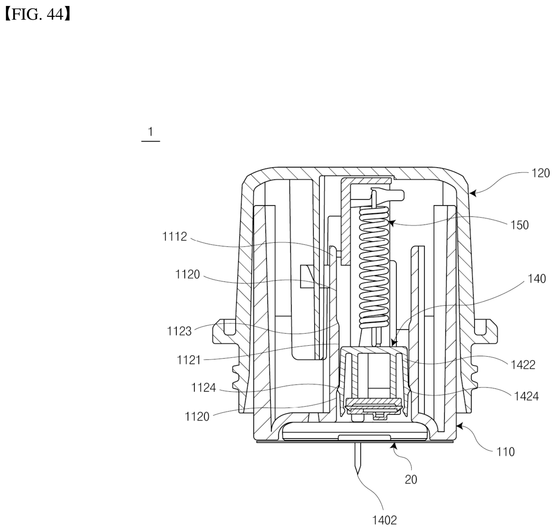

Thereafter, as illustrated in FIG. 44, the grip arm protrusion portion 1424 passes through the second step inclined surface 1124 so that the pressing of the grip arm protrusion portion 1424 by the step portion 1121 may be released, and the grip arm 1422 may grip the sensor unit 30 in a somewhat loose state so as to deliver the sensor unit 30 to the transmission unit 40. In the insertion process of the transcutaneous sensor member 330 into the subcutaneous tissue, since the sensor unit 30 continuously receives the force in the first direction by the sensor unit housing 310, even if the grip arm 1422 grips the sensor unit 30 in a somewhat loose state to deliver the sensor unit 30 to the transmission unit 40, the sensor unit 30 may be accurately delivered to the seating groove 412. Meanwhile, since the grip arm 1422 grips the sensor unit 30 in a somewhat loose state to deliver the sensor unit 30 to the seating groove 412 of the transmission unit 40, an operational error that occurs in which the sensor unit 30 is brought along with the needle carrier 140, in a process in which the needle carrier 140 returns to a retracted position, can be effectively prevented.

### Exemplary embodiment of operation of transmission unit support portion

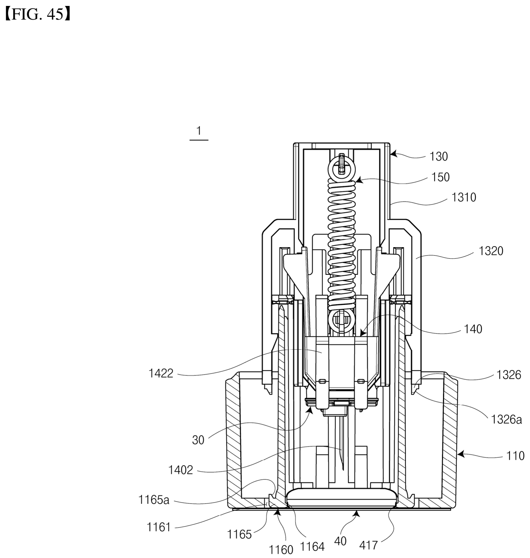

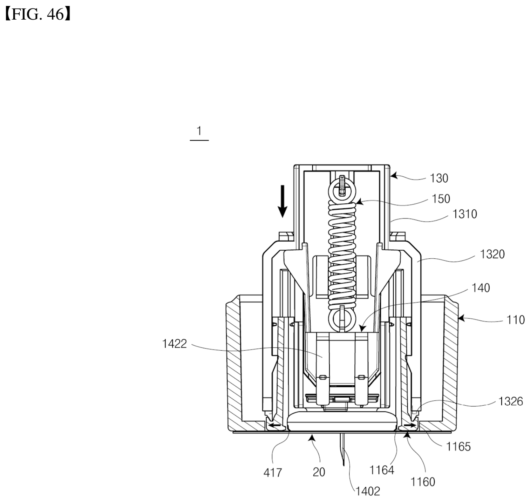

FIGS. 45 and 46 are sequentially illustrated cross-sectional views for explaining an exemplary embodiment of movement constraint and movement constraint release of the transmission unit 40 by the transmission unit support portion 1160 during an operation process of the applicator assembly 1.

As illustrated in FIG. 45, in a state before operation of the applicator assembly 1, a leading end portion of the catching portion 1164 provided in the transmission unit support hook 1162 may be disposed in an interior of the transmission unit housing groove 417 of the transmission unit housing 410 so as to effectively prevent the transmission unit 40 from being arbitrarily deviated from the transmission unit accommodation portion 1104. In this case, the extension arm pushing portion 1326 provided in the sensor unit carrier 130 may maintain a state spaced apart from the pushed portion 1165 of the transmission unit support hook 1162. Since the transmission unit 40 is prevented by the transmission unit support hook 1162 from being arbitrarily deviated from the transmission unit accommodation portion 1104 before operation of the applicator assembly 1 or in the insertion process of the transcutaneous sensor member 330 into the subcutaneous tissue, the economic efficiency and operational accuracy of the applicator assembly 1 can be effectively improved.

Meanwhile, as illustrated in FIG. 46, when the applicator assembly 1 operates and the sensor unit carrier 130 moves to an insertion position, the extension arm pushing portion 1326 presses the inclined surface 1165a formed on the pushed portion 1165 so that the transmission unit support hook 1162 moves while being deformed in a direction away from the transmission unit 40, and accordingly, the leading end portion of the catching portion 1164 may be deviated from the transmission unit housing groove 417, thereby releasing the movement constraint of the transmission unit 40 by the transmission unit support hook 1162. The transmission unit support portion 1160, through interaction with the extension arm pushing portion 1326, releases the movement constraint of the transmission unit 40 by the transmission unit support hook 1162 in the insertion process of the transcutaneous sensor member 330 into the subcutaneous tissue, thereby effectively preventing a phenomenon in which the wearable unit 20 is brought along with the applicator 10 in a process of separating the wearable unit 20 from the applicator 10 after completion of the insertion of the transcutaneous sensor member 330 into the subcutaneous tissue.

Although the present invention has been described in detail through the embodiments above, other forms of embodiments are also possible. Therefore, the technical teachings and scope of the claims described below are not limited to the embodiments.

### [Description of Reference Numerals]

| | | | |
|---|---|---|---|
| 1: | Applicator assembly | 5: | External terminal |
| 10: | Applicator | 20: | Wearable unit |
| 30: | Sensor unit | 40: | Transmission unit |
| 50: | Cap | 110: | Body housing |
| 120: | Handle housing | 130: | Sensor unit carrier |
| 140: | Needle carrier | 150: | Elastic member |

## Claims

1. An applicator, comprising:
a body housing having a movement space formed therein;
a sensor unit carrier provided to be movable in the movement space;
a bridge pressing portion disposed on one of the body housing and the sensor unit carrier; and
a bridge provided on the other one of the body housing and the sensor unit carrier and disposed at a position corresponding to the bridge pressing portion,
wherein the bridge is configured to restrict movement of the sensor unit carrier from an initial position to an insertion position by contacting with the bridge pressing portion and blocking movement of the bridge pressing portion.

2. The applicator of claim 1, wherein the body housing comprises a column disposed to form the movement space in the interior of the body housing and provided with the bridge pressing portion at an end portion thereof, and
wherein the sensor unit carrier comprises:
a sensor unit carrier body of which at least a portion is accommodated in the movement space and is provided to move along the movement space;
an extension arm extending from a side end of the sensor unit carrier body and disposed at an outside of the column; and
the bridge having one end connected to the sensor unit carrier body and the other end connected to the extension arm, and disposed at a position facing the bridge pressing portion.

3. The applicator of claim 1 or 2, wherein when the sensor unit carrier is positioned at the initial position, the bridge is in contact with the bridge pressing portion or is positioned at a position spaced apart from the bridge pressing portion.

4. The applicator of any one of the preceding claims, wherein the sensor unit carrier moves in the movement space along a first direction from the initial position to the insertion position, and when the sensor unit carrier is positioned at the insertion position, at least a portion of the bridge is positioned at a position overlapping at least a portion of the bridge pressing portion.

5. An applicator, comprising:
a body housing having an interior in which a movement space is formed along a first direction;
a sensor unit carrier provided to be relatively movable with respect to the body housing in the movement space;
a bridge pressing portion provided in one of the body housing or the sensor unit carrier; and
a bridge configured to restrict movement of the bridge pressing portion and disposed at a position contactable with the bridge pressing portion in the other one of the body housing or the sensor unit carrier,
wherein movement restriction of the sensor unit carrier in the first direction is released by shape deformation of the bridge due to pressing of the bridge pressing portion.

6. The applicator of claim 5, wherein the bridge comprises a vulnerable portion in which shape deformation is concentrated compared to other regions when pressure is applied by the bridge pressing portion.

7. The applicator of claim 6, wherein the body housing comprises a column disposed to form the movement space in the interior of the body housing and provided with the bridge pressing portion at an end portion thereof,
wherein the sensor unit carrier comprises:
a sensor unit carrier body of which at least a portion is accommodated in the movement space and is provided to move along the movement space;
an extension arm extending from a side end of the sensor unit carrier body and disposed at an outside of the column; and
the bridge having one end connected to the sensor unit carrier body and the other end connected to the extension arm, and disposed at a position facing the bridge pressing portion, and
wherein the vulnerable portion is formed at a position closer to one surface of the sensor unit carrier body to which the bridge is connected compared to a leading end portion of the bridge pressing portion.

8. The applicator of claim 7, wherein a carrier slit formed by incision along the first direction is provided at a leading end portion of the column adjacent to the bridge pressing portion,
wherein an extension arm guide groove is formed to be recessed along the first direction in a shape corresponding to the carrier slit on one surface of an extension arm connection portion disposed between the sensor unit carrier body and the extension arm, and
wherein as the sensor unit carrier moves along the first direction, the extension arm guide groove is introduced into the carrier slit so that movement of the sensor unit carrier in the first direction is guided.

9. The applicator of any one of claims 6 to 8, wherein the vulnerable portion is formed to have a smaller thickness compared to the bridge.

10. The applicator of any one of claims 5 to 9, wherein the bridge is disposed in a direction intersecting the first direction, and the bridge pressing portion is disposed along the first direction.

11. The applicator of any one of claims 7 to 10, wherein the bridge pressing portion is provided with at least one inclined surface and is disposed at the end portion of the column in a shape in which a cross-section decreases toward the bridge, and/or
wherein the leading end portion of the bridge pressing portion has a shape in which a cross-section decreases in a direction opposite to the first direction.

12. The applicator of any one of claims 5 to 11, wherein movement restriction of the sensor unit carrier in the first direction is released by cutting of the bridge.

13. The applicator of any one of claim 5 to 12, further comprising:
a handle housing connected to the body housing so that one end thereof is in contact with the sensor unit carrier and moves together with the sensor unit carrier,
wherein movement restriction of the handle housing in the first direction is released only when the bridge is deformed by application of a pressure exceeding a reference pressure to the handle housing.

14. An applicator assembly, comprising:
a transmission unit intended to be delivered to a detection position on skin;
a body housing having an interior in which a movement space is formed along a first direction, the transmission unit being separably fixed to one end thereof;
a sensor unit carrier provided to move along the movement space;
a sensor unit comprising a transcutaneous sensor member capable of detecting biometric information in a subcutaneous tissue of a human body, and provided to move together with the sensor unit carrier in the movement space along the first direction to be coupled to the transmission unit;
a bridge pressing portion provided in one of the body housing or the sensor unit carrier; and
a bridge configured to restrict movement of the sensor unit carrier and disposed at a position corresponding to the bridge pressing portion in the other one of the body housing or the sensor unit carrier, and provided to be cut only when a pressure applied from the bridge pressing portion exceeds a reference pressure,
wherein movement restriction of the sensor unit carrier in the first direction is released by cutting of the bridge.

15. The applicator assembly of claim 14, wherein the bridge is provided in a shape extending along a direction intersecting the first direction, and
wherein the bridge pressing portion is disposed along a direction parallel to the first direction.
